# EUROPEAN PATENT SPECIFICATION

(11) **EP 3 841 218 B1**
(45) Date of publication and mention of the grant of the patent: **26.06.2024**
(21) Application number: 19759021.9
(22) Date of filing: 22.08.2019
(51) Int. Cl.: C12Q 1/6834

(54) **NUCLEIC ACID BASED DETECTION METHODS**
VERFAHREN ZUR DETEKTION AUF DER BASIS VON NUKLEINSÄUREN
PROCÉDÉS DE DÉTECTION REPOSANT SUR UN ACIDE NUCLÉIQUE

(30) Priority: 23.08.2018 GB 201813789
(43) Date of publication of application: 30.06.2021
(73) Proprietor: Aptamer Diagnostics Limited, Heslington York North Yorkshire YO10 5NY (GB)
(72) Inventor: TOLLEY, Arron Craig, York, Yorkshire YO10 5NY (GB); BUNKA, David Harry John, York, Yorkshire YO10 5NY (GB)
(74) Representative: Secerna LLP
(86) International application number: PCT/GB2019/052361
(87) International publication number: WO 2020/039201

(56) References cited:
- WO-A1-99/31275
- WO-A1-2007/109500
- CHUNFENG WANG ET AL: "Development Of A Nucleic Acid Lateral Flow Strip For Detection Of Hepatitis C Virus (Hcv) Core Antigen", NUCLEOSIDES, NUCLEOTIDES AND NUCLEIC ACIDS., vol. 32, no. 2, 1 January 2013 (2013-01-01), pages 59-68, XP055543334, US ISSN: 1525-7770, DOI: 10.1080/15257770.2013.763976
- SHENG GONG ET AL: "Immunochromatographic strip biosensor for the rapid detection of N-glycolylneuraminic acid based on aptamer-conjugated nanoparticle", ANALYTICAL BIOCHEMISTRY, vol. 561-562, 20 July 2018 (2018-07-20), pages 52-58, XP055543627, AMSTERDAM, NL ISSN: 0003-2697, DOI: 10.1016/j.ab.2018.07.017
- AILIANG CHEN ET AL: "Replacing antibodies with aptamers in lateral flow immunoassay", BIOSENSORS AND BIOELECTRONICS, vol. 71, 14 April 2015 (2015-04-14), pages 230-242, XP055439702, AMSTERDAM, NL ISSN: 0956-5663, DOI: 10.1016/j.bios.2015.04.041

## Description

### Field of the Invention

Embodiments of the present invention relate to apparatus and methods of detecting and/or quantifying a target moiety in a sample comprising the use of a nucleic-acid molecule based recapture event. Particularly although not exclusively certain embodiments of the present invention relate to apparatus and assays which comprise a displacement of an immobilised nucleic acid molecule to form a target-nucleic acid molecule and detection of a subsequent recapture event of either the target-nucleic acid complex or the nucleic acid molecule on its own. Other aspects and embodiments are described herein.

### Background to the Invention

Aptamers are small artificial ligands, including single stranded DNA, RNA or polypeptide molecules which are capable of binding to specific target moieties of interest with high affinity and specificity. Aptamers are regarded as alternatives to antibodies for use as diagnostic agents and/or therapeutic agents. Furthermore, aptamers have also shown promise as antibody replacements in many analytical applications in the fields of environmental and food research, as well as clinical diagnostics.

Currently, aptamers are obtained by an iterative process of screening of a partially randomised library of candidate oligonucleotides by an affinity-based partitioning against the target moiety of interest. Aptamers have high structural stability over a wide range of pH and temperatures making them ideal reagents for a broad spectrum of *in-vitro, in-vivo* and *ex-vivo* applications.

In 1990, an *in-vitro* method that mimics the evolutionary process was developed to identify high-affinity aptamers, by two independent groups Tuerk & Gold, 1990 (Science 249, 505-510) and Ellington & Szostak 1990 (Nature 346, 818-822)). The process is referred to as *in-vitro* selection. The process exploits fundamental concepts of evolution, utilising variation, selection, and replication to achieve high target affinity and specificity from a starting pool of degenerate nucleic acid molecules (i.e., oligonucleotides). Typically, for selection of nucleic acid (DNA or RNA oligonucleotides) aptamers, variation is achieved by synthesising a degenerate library of short oligonucleotides (about ~10¹⁴ different sequences), ranging in size from about 20 to about 100 nucleotides. Each oligonucleotide generally comprises an internal random region flanked by primer regions for subsequent amplification by suitable amplification reaction, e.g., the polymerase chain reaction (PCR) for DNA, or reverse transcription polymerase chain reaction (RT-PCR) followed by *in vitro* transcription reactions for RNA.

Current methods of aptamer selection involve incubating the nucleic acid pool with the immobilised target molecule(s) under conditions which allow binding of nucleic acid molecules to the target molecules; followed by extensive washing to remove non-binding or low affinity sequences. The bound nucleic acid molecules are eluted and amplified using PCR or other amplification method. The amplified nucleic acid molecules then form the starting material input for the next round of *in-vitro* selection. This process is repeated multiple times until one or more nucleic acid aptamers are enriched, which bind tightly and specifically to the target molecule(s). Recent technical advances in the preparation of random libraries and in affinity-based partitioning methods have resulted in aptamers with equivalent affinities to antibodies.

Aptamers have been raised against a diverse range of target molecules including for example inorganic components, small organic molecules, proteins, peptides, nucleic acids, carbohydrates and cells. As such, aptamers can be used to detect and also quantify amount of a specific product in a given sample.

There are several advantages of aptamers over antibodies including for example: ease of *in-vitro* synthesis, flexible modification, broad target ranges, reusability, and high thermal/chemical stability. As a result, aptamers are used in place of antibodies in a number of assays and detection methods. In one example, aptamers are now being used in place of antibodies in assays such as ELISA (enzyme-linked immunosorbent assay). When aptamers are used in place of antibodies, the resulting assay is often referred to as an "ELONA" (enzyme-linked oligonucleotide assay), "ELASA" (enzyme linked aptamer sorbent assay), "ELAA" (enzyme-linked aptamer assay) or similar. Incorporating aptamers into these ELISA-like assay platforms can result in increased sensitivity, allow a greater number of analytes to be detected; including analytes for which there are no antibodies available and a wider range of outputs, since aptamers can be conjugated to multiple reporter molecules including fluorophores and quencher molecules. Other disadvantages associated with the use of antibodies include for example inappropriate aggregation and complications associated with labelling and/or production of antibodies.

Aptamers have also been used in a number of other assay formats, such as lateral flow devices, biosensors and similar assay formats. For example, Wang et al 2013 (Nucleosides, Nucleotides and Nucleic acids, 32, 2, 59-68) relates to a nucleic acid lateral flow strip for detection of HCV core antigen. Gong et al 2018 (Analytical Biochemistry, 561, 52-58) relates to an immunochromatographic strip biosensor based on aptamer-conjugated nanoparticle. WO2007/1 09500A1 relates a lateral flow device. However, there remains a need to provide assays which incorporate aptamers with good sensitivity and accuracy.

Embodiments of the present invention aim to overcome the problems associated with the prior art.

### Summary of Certain Embodiments of the invention

The present invention relates to assays that utilize the combination of displacement of nucleic acid molecules (e.g. aptamers) from a support and subsequent recapture of said nucleic acid molecules by hybridization to complementary oligonucleotides (e.g. capture oligonucleotides).

Advantageously, higher amounts of a target molecule may lead to displacement of more of the nucleic acid molecules from the support. In turn, this may lead to a greater amount of nucleic acid molecules that are recaptured. As such, certain embodiments of the invention provide highly sensitive and accurate assays that utilize a concentration-dependent and/or gain of signal format.

Advantageously, the capture oligonucleotides may be configured to hybridize to nucleic acid sequences common to any aptamer obtained from the "universal aptamer selection library" as described herein. As such, certain embodiments of the invention provide universal assays allowing substitution of aptamers against different target molecules with no or minimal re-optimization. Various aspects and embodiments are described herein.

In a first aspect of the present invention, there is provided an apparatus for detecting the presence, absence or level of a target molecule in a sample, the apparatus comprising:
a) a first region comprising:
   i) a support; and
   ii) an immobilisation oligonucleotide; and
   iii) a nucleic acid molecule which has a binding affinity to a target molecule, wherein the nucleic acid molecule is configured to form a complex with the target molecule,
   wherein the immobilisation oligonucleotide is attached directly or indirectly to the support, and further wherein the immobilisation oligonucleotide comprises a nucleic acid sequence which is at least partially complementary to a nucleic acid sequence of the nucleic acid molecule and wherein the nucleic acid molecule is capable of hybridizing to the immobilisation oligonucleotide and further wherein the nucleic acid molecule is capable of being displaced from the complex with the immobilisation oligonucleotide if the target molecule is present in the sample; the apparatus further comprising:
b) a further region comprising:
   i) a support; and
   ii) a capture oligonucleotide attached directly or indirectly to the support, wherein the capture oligonucleotide comprises a nucleic acid sequence which is at least partially complementary toa nucleic acid sequence of the nucleic acid molecule, wherein the capture oligonucleotide is configured to hybridize to the nucleic acid sequence of the nucleic acid molecule when in complex with the target molecule to capture the nucleic acid-molecule-target molecule complex thereby enabling the presence or level of the target molecule to be detected.

Thus, in certain embodiments, the apparatus utilises a nucleic acid molecule which is configured to bind to a target molecule. Aptly, the nucleic acid molecule binds with high affinity and specificity to the target molecule.

Aptly, in the absence of the target molecule, the nucleic acid molecule is secured directly or indirectly to a support to immobilise it. The nucleic acid molecule may be attached by way of hybridizing to an immobilisation oligonucleotide which is in turn directly or indirectly attached to the support.

Certain embodiments of the present invention utilise the ability of a nucleic acid molecule e.g. an aptamer, to change conformation when it binds to its target molecule. The conformational change causes the nucleic acid molecule to disassociate from the immobilisation oligonucleotide thus releasing the nucleic acid molecule in complex with the target molecule. If the target molecule is not present, the nucleic acid molecule does not undergo the conformation change and as such remains hybridized to the immobilisation oligonucleotide.

Aptly, a linker molecule is attached to the support and wherein the linker molecule is configured to hybridize to the immobilisation oligonucleotide and further wherein the nucleic acid molecule is configured to hybridize to the immobilisation oligonucleotide when the immobilisation oligonucleotide is hybridized to the linker molecule.

Furthermore, certain embodiments of the present invention utilise a "re-capture" event in which the nucleic acid molecule when in complex with the target molecule is captured by a capture oligonucleotide. The recapture event can then enable the presence of the target molecule and optionally also the amount of the target molecule to be detected.

In certain embodiments, the capture oligonucleotide comprises a nucleic acid sequence which is at least partially complementary to a nucleic acid sequence of the nucleic acid molecule, wherein the capture oligonucleotide is configured to hybridize to the nucleic acid sequence of the nucleic acid molecule when in complex with the target molecule to capture the nucleic acid-molecule-target molecule complex.

In certain embodiments, the nucleic acid molecule comprises at least one fixed nucleic acid sequence region. The fixed nucleic acid sequence region may be a primer region. In certain embodiments, the capture oligonucleotide comprises a nucleic acid sequence which is complementary to the fixed nucleic acid sequence region of the nucleic acid molecule.

In certain embodiments, the fixed nucleic acid sequence region may be a sequence which is universally used in the design of an aptamer library such that every aptamer which is selected from the library comprises the fixed nucleic acid sequence. Thus, apparatus can be designed with a capture oligonucleotide which is capable of hybridizing to multiple aptamers, each aptamer having one or more regions which have the same predetermined nucleic acid sequence whilst also being specific to a different target molecule.

In certain embodiments, the nucleic acid molecule comprises a first fixed nucleic acid sequence region and a second nucleic acid sequence region. Thus, the first nucleic acid molecule may be a forward primer and the second nucleic acid molecule may be a reverse primer. In certain embodiments, the first fixed nucleic acid sequence region is located at a 5' end region of the nucleic acid molecule and the second fixed nucleic acid sequence region is located at a 3' end region of the nucleic acid molecule.

In certain embodiments, the capture oligonucleotide is capable of hybridizing to either the first fixed nucleic acid sequence region or a part thereof, or the second fixed nucleic acid sequence region or a part thereof.

In certain embodiments, the apparatus further comprises a detection means for detecting formation of a complex comprising the capture oligonucleotide and the nucleic acid molecule.

In certain embodiments, the linker molecule is a DNA or an RNA molecule or a mixed DNA/RNA molecule, wherein optionally the linker molecule comprises one or more modified nucleotides.

In certain embodiments, the apparatus comprises a lateral flow assay device. Aptly, the first region comprises a sample receiving region. In certain embodiments, the apparatus further comprises a flow path between the first region and the further region.

In certain embodiments the flow path comprises a membrane, e.g. a nitrocellulose, a polyethylene (PE), a polytetrafluoroethylene (PTFE), a polypropylene (PP), a cellulose acetate (CA), a polyacrylonitrile (PAN), a polyimide (PI), a polysulfone (PS), a polyethersulfone (PES) membrane or an inorganic membrane comprising aluminum oxide (Al2O3), silicon oxide (SiO2) and/or zirconium oxide (ZrO2).

In certain embodiments, the first region comprises a sample application zone, wherein optionally the sample application zone comprises a sample application pad. In certain embodiments, the support of the first region comprises an immobilisation zone which comprises the immobilisation oligonucleotide directly or indirectly attached.

In certain embodiments, the immobilisation zone comprises non-woven fibers e.g. cellulose fibers, glass fibers, silicon carbide fibers, polymer fibers, animal fibers (e.g. wool, silk), carbon fibers, mineral fibers, and/or micro fibers. In certain embodiments the immobilisation zone comprises woven fibers.

In certain embodiments, the first region is provided upstream from the further region. In certain embodiments, the further region comprises a capture zone in which the capture oligonucleotide is indirectly or directly attached to the support.

In certain embodiments, the further region comprises a plurality of capture zones, each capture zone comprising a capture oligonucleotide directly or indirectly attached to the support, wherein each capture oligonucleotide may be the same or may be different.

In certain embodiments, the apparatus further comprises a control zone located downstream from the first region, wherein the control zone comprises a further capture oligonucleotide. In certain embodiments, the apparatus comprises a plurality of control zones, each control zone comprising a capture oligonucleotide, wherein each capture oligonucleotide is the same or different.

Aptly, each capture oligonucleotide in respective control zones is configured to bind to a different molecule.

In certain embodiments, the apparatus may comprise a plurality of nucleic acid molecules, each nucleic acid molecule being specific for a different target molecule. In certain embodiments, each nucleic acid molecule comprises one or more e.g. 2 fixed nucleic acid sequence regions which are the same for more than one nucleic acid molecule. In certain embodiments, each nucleic acid molecule comprises the same fixed nucleic acid sequence region(s) which allows the same capture oligonucleotide to be used to capture every nucleic acid molecule regardless of the nature of the target molecule. Each nucleic acid molecule may comprise a different detection means to enable separate detection and such that the apparatus may be suitable for use in a multiplex assay format.

In certain embodiments, the apparatus further comprises a housing enclosing the first region and the further region. The housing may further comprise a sample introduction port.

In certain embodiments, the housing further comprises a window adjacent to the detection zone and optionally the control zone. In certain embodiments, the support of the first region and/ or the further region is independently selected from a bead, a microtiter or other assay plate, a strip, a membrane, a film, a gel, a chip, a microparticle, a nanoparticle, a nanofiber, a nanotube, a micelle, a micropore, a nanopore, and a biosensor surface.

In certain embodiments, the first region is comprised in a first vessel and the further region is comprised in a further vessel. Aptly, the solid phase of the first region and the further region are comprised on a strip. Aptly, the strip further comprises a flow path between the first region and the further region.

In certain embodiments the apparatus further comprises one or more of the following:
a) an absorbance pad,
b) a membrane, e.g. a nitrocellulose membrane;
c) one or more competitor molecules; and
d) one or more control molecules.

In certain embodiments, the apparatus further comprises a vessel configured to accommodate at least one end portion of the strip and the sample. In the vessel comprises a sample introduction region. In certain embodiments, the immobilisation oligonucleotide and/or the capture oligonucleotide is selected from a DNA molecule, a RNA molecule, a mixed DNA/RNA molecule, a modified DNA molecule and a modified RNA molecule.

In certain embodiments, the apparatus comprises a plurality of immobilisation oligonucleotides, a plurality of capture oligonucleotides and a plurality of nucleic acid molecules.

In certain embodiments, the nucleic acid molecule is an aptamer. Aptly, the aptamer is selected from a double-stranded aptamer, a single-stranded aptamer and an aptamer which is double-stranded over at least a portion of its length. In certain embodiments, the nucleic acid molecule and/or the immobilisation molecule comprises a detectable label.

In certain embodiments, the detectable label is selected from a fluorophore, a nanoparticle, a quantum dot, an enzyme, a radioactive isotope, a pre-defined sequence portion, a biotin, a desthiobiotin, a thiol group, an amine group, an azide, an aminoallyl group, a digoxigenin, an antibody, a catalyst, a colloidal metallic particle, a colloidal non-metallic particle, an organic polymer, a latex particle, a nanofiber, a nanotube, a dendrimer, a protein, and a liposome.

In certain embodiments, the detectable label is an enzyme, wherein optionally the enzyme is selected from horseradish peroxidase, alkaline phosphatase, urease and β-galactosidase.

In certain embodiments, the sample is a biological sample selected from whole blood, leukocytes, peripheral blood mononuclear cells, plasma, serum, sputum, breath, urine, semen, saliva, meningial fluid, amniotic fluid, glandular fluid, lymph fluid, nipple aspirate, bronchial aspirate, synovial fluid, joint aspirate, cells, a cellular extract, stool, tissue, a tissue biopsy, and cerebrospinal fluid.

In certain embodiments, the sample is derived from an agricultural or industrial product or by-product, an environmental sample, water, a food product, a sample of a production process, an animal product, a plant product and a bacterial product.

In certain embodiments, the target molecule is selected from an organic or inorganic small molecule, a cell, a protein, a peptide, an amino acid, a carbohydrate, a lipid, a virus, a microorganism, a tissue section, an ion, a nucleotide, a nucleotide derivative and a nucleic acid.

In a further aspect of the present invention there is provided a method of detecting the presence, absence or amount of a target molecule in a sample, the method comprising:
a) interacting a sample with a complex comprising:
   i) an immobilisation oligonucleotide; and
   ii) a nucleic acid molecule, wherein the immobilisation oligonucleotide is attached directly or indirectly to a support, the immobilisation oligonucleotide comprising a nucleic acid sequence which is at least partially complementary to a nucleic acid sequence of the nucleic acid molecule and wherein the nucleic acid molecule is capable of hybridizing to a portion of the immobilisation oligonucleotide;
   wherein the nucleic acid molecule has a binding affinity to a target molecule, and further wherein the nucleic acid molecule is configured to form a complex with the target molecule;
b) if the target molecule is present in the sample, disassociating the nucleic acid molecule from the complex with the immobilisation oligonucleotide to form a target molecule-nucleic acid molecule complex; and
c) providing a capture oligonucleotide which comprises a nucleic acid sequence which is at least partially complementary to a nucleic acid sequence of the nucleic acid molecule, wherein the nucleic acid molecule is capable of hybridizing to a portion of the capture oligonucleotide; and
d) detecting the presence or absence of the target molecule.

In certain embodiments, the method further comprises, if a target molecule is present in the sample, forming a target molecule-nucleic acid molecule-capture oligonucleotide complex. In certain embodiments, the method further comprises quantifying the amount of target molecule in the sample.

In certain embodiments, detection of the target molecule comprises photonic detection, electronic detection, acoustic detection, electro-chemical detection, electro-optic detection, enzymatic detection, chemical detection, biochemical detection or physical detection.

In certain embodiments, step (a) is performed under conditions effective to allow binding between the target molecule and the nucleic acid molecule. In certain embodiments, the step of detecting the presence or absence of the target molecule comprises detecting the hybridization of the nucleic acid molecule to the capture oligonucleotide.

In certain embodiments, the method comprises bio-layer interferometry (BLI). In certain embodiments, the method comprises a step of determining displacement of a nucleic acid molecule from a nucleic acid molecule- immobilisation oligonucleotide complex. In certain embodiments, the method further comprises detecting hybridization of a nucleic acid molecule-target molecule complex to a capture oligonucleotide. In certain embodiments, the method comprises incubating the nucleic acid molecule-target molecule complex and immobilized capture oligonucleotide for at least 2 minutes, e.g. 3 minutes, 4 minutes, 5 minutes of more prior to the step of detecting the hybridization of a nucleic acid molecule-target molecule complex to the capture oligonucleotide. In certain embodiments, the capture oligonucleotide is immobilised e.g. to a surface of a BLI probe.

In certain embodiments, the method comprises surface plasmon resonance (SPR). Certain embodiments of the present invention may provide the advantage of detection of small molecules which cannot usually be detected. Particularly, using systems such as BLI (which might not normally be sensitive enough to detect), certain embodiments of the present invention involve the displacement of nucleic acid molecules from an immobilisation oligonucleotide which gives a much greater signal than a signal generated by the binding of a small molecule.

### Detailed Description of Certain Embodiments of the invention

### Brief Description of the Figures

Certain embodiments of the present invention will be described in more detail below, with reference to the accompanying Figures in which:

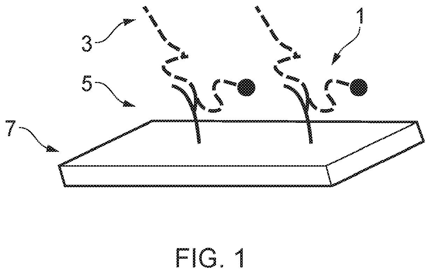

Figure 1 is a schematic representation of a first region of an apparatus according to certain embodiments of the present invention;
Figure 2 is a schematic representation of an apparatus according to certain embodiments of the present invention;
Figure 3 is a schematic representation of a nucleic acid molecule for use in the apparatus and methods according to certain embodiments of the present invention. Particularly, the nucleic acid molecule comprises at least one fixed 'capture sequence' (black bar) which is complementary to an immobilization oligonucleotide and/or a "capture oligonucleotide" sequence. These sequences are used to immobilise the nucleic acid molecule, whether alone or in complex with a target molecule, and/or to hybridise with the capture oligonucleotide in the "re-capture" event.
   In certain embodiments, the 'immobilisation oligonucleotide' has a linker (dotted line) to reduce steric hindrance from the first and/or further support. Aptly, the nucleic acid molecule also comprises 2 fixed 'primer sequences' (black bars) which are used to amplify the library by PCR etc. One (or both) of these primer regions may be modified with a fluorophore (circle) or other functional moiety (nanoparticle, quantum dot, enzyme etc) to allow detection of the nucleic acid molecule as required in the assay. The primer regions can also be used as "capture regions" to hybridise with the capture oligonucleotide in the "re-capture" event.

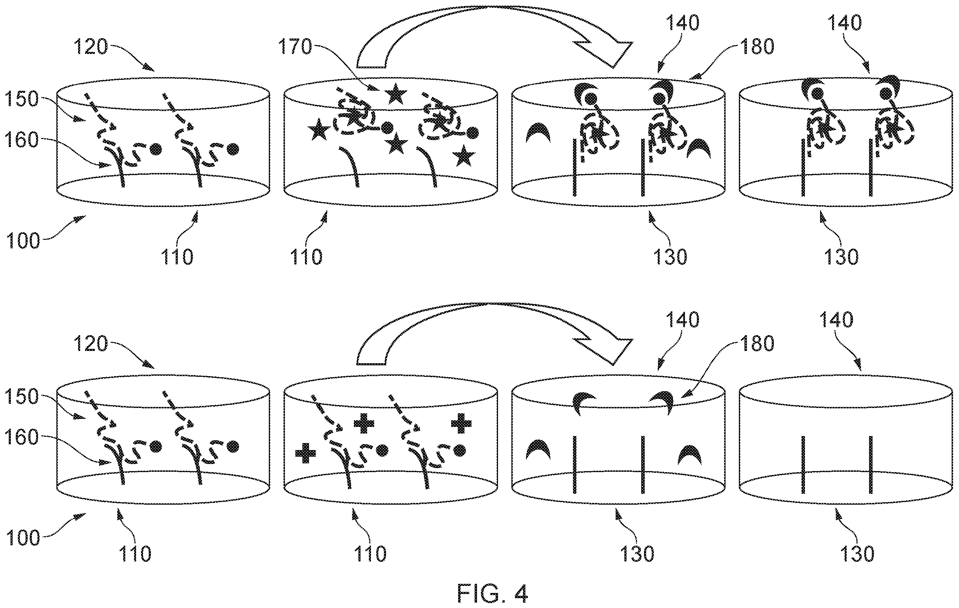

Figure 4 is a schematic representation of an apparatus and method according to certain embodiments of the present invention in which an ELONA assay is performed.

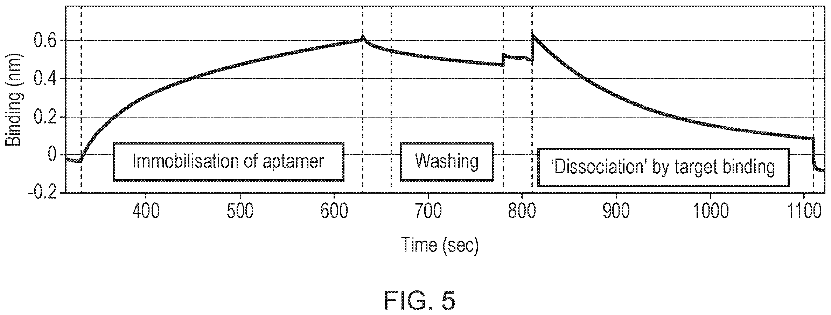

Figure 5 is a graph illustrating BioLayer Interferometry response curves at different steps of the Aptamer Displacement Assay according to certain embodiments of the present invention. A first aptamer was immobilised onto the streptavidin-coated probe via a biotinylated complementary immobilisation oligonucleotide. After several washing steps, the probe was incubated with the target molecule. Target binding causes an aptamer displacement seen as 'dissociation' signal.

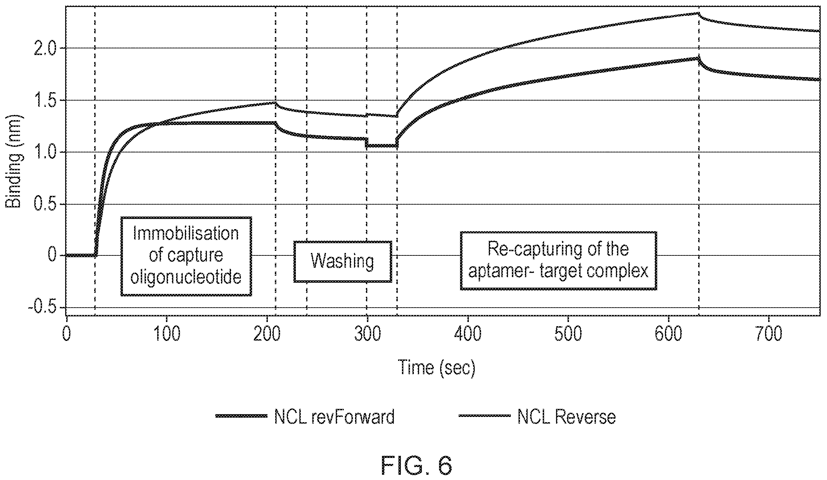

Figure 6 provides data showing BioLayer Interferometry response curves at different steps of the aptamer re-capture assay method. Firstly, the biotinylated capture oligonucleotides revFOR (green) and REV (pink) were immobilised onto the streptavidin-coated probes, respectively. After several washing steps, the probes were incubated with the aptamer-target complexes obtained from the aptamer-displacement assay in Figure 5. Aptamer re-capturing is seen as an increasing signal as the aptamer-target complex binds to the immobilised capture oligonucleotide. Figure 6 indicates that either fixed primer region can be used to recapture the aptamer-target molecule complex.

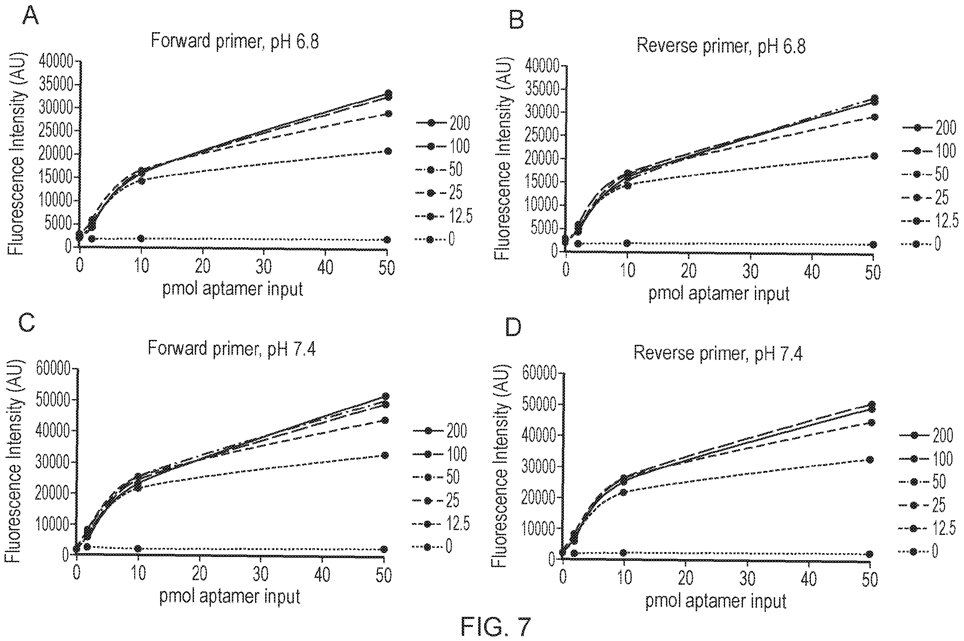

Figure 7 A to D show ELISA-like assays demonstrating concentration dependent recapture. Each of panels A to D show fluorescence from the recaptured (FAM labelled) aptamers (y-axis), against the amount of input aptamer (x-axis) incubated with pre-immobilised plate with 'capture oligo'. When no 'capture oligo' is present (`0', dotted line), no aptamer is re-captured. As the amount of 'capture oligo' is increased, more aptamer is re-captured and gives more fluorescence. Pre-immobilising the ELISA plate with biotinylated 'capture oligo' allows recapture of aptamers in a concentration dependent manner. The aptamers can be recaptured by either the 5' or 3' ends (forward and reverse on charts A & C, and B & D respectively). The data also shows that the aptamers can be recaptured at either of the common buffer pH used in the displacement assays; pH6.8 (A & B) and pH7.4 (C & D).

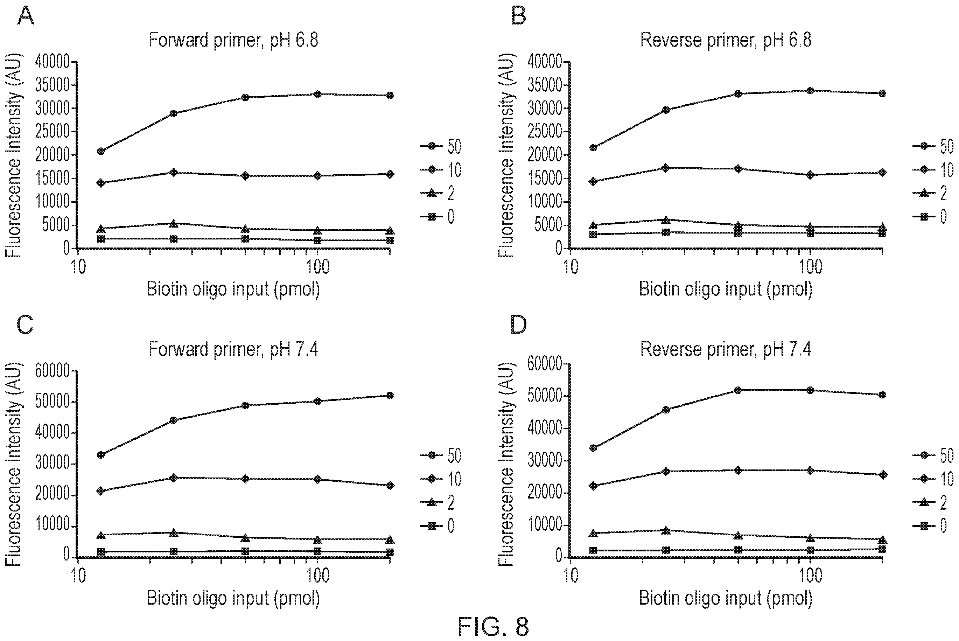

Figure 8 A to D also show ELISA-like assays demonstrating concentration dependent recapture. Each of panels A to D show fluorescence from the recaptured (FAM labelled) aptamers (y-axis), against the amount of 'capture oligo' incubated with the plate (x-axis). Each series (circles, diamonds, triangles and squares) is a different amount of fluorescently labelled aptamer (50, 10, 2, 0 pmol of aptamer respectively). At the highest aptamer amount (50pmol, circles) a clear trend can be seen in each of the buffers and with both 'capture oligos'. This shows that either 'end' of the aptamer (5' in A & C or 3' in B & D) can be used to recapture, and that the buffer pH does not have a significant impact on binding. Concentration dependent recapture is seen with increasing amount of biotinylated capture oligo pre-immobilised on the plate. Saturation is reached when the amount of aptamer reaches the same amount as the pre-immobilised biotinylated capture oligo.

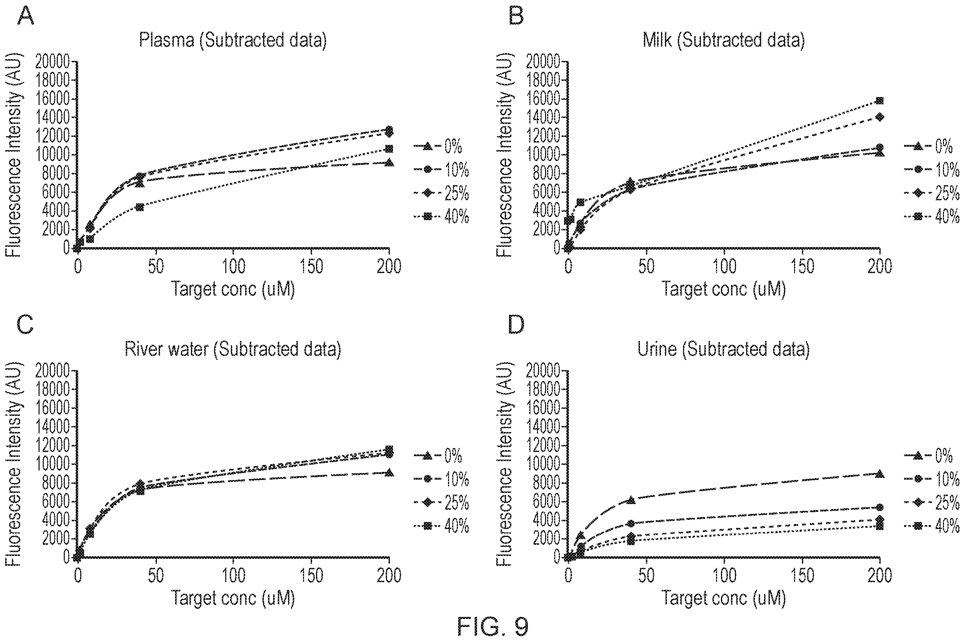

Figure 9 A to D shows results of an ELONA-like assay. It illustrates measured fluorescence signals representing the amount of an aptamer displaced from a surface upon binding to a small molecule target, at 4 different concentrations of test substrates/matrices (human plasma, milk, river water and mock urine, at concentrations of 0%, 10%, 25%, 40% v/v, respectively). The data was plotted after being corrected by subtracting the fluorescence from the '0 µM Target Molecule' control wells. Concentration dependant binding is seen between the target molecule and the aptamer at 4 different concentrations of tested substrates/matrices;

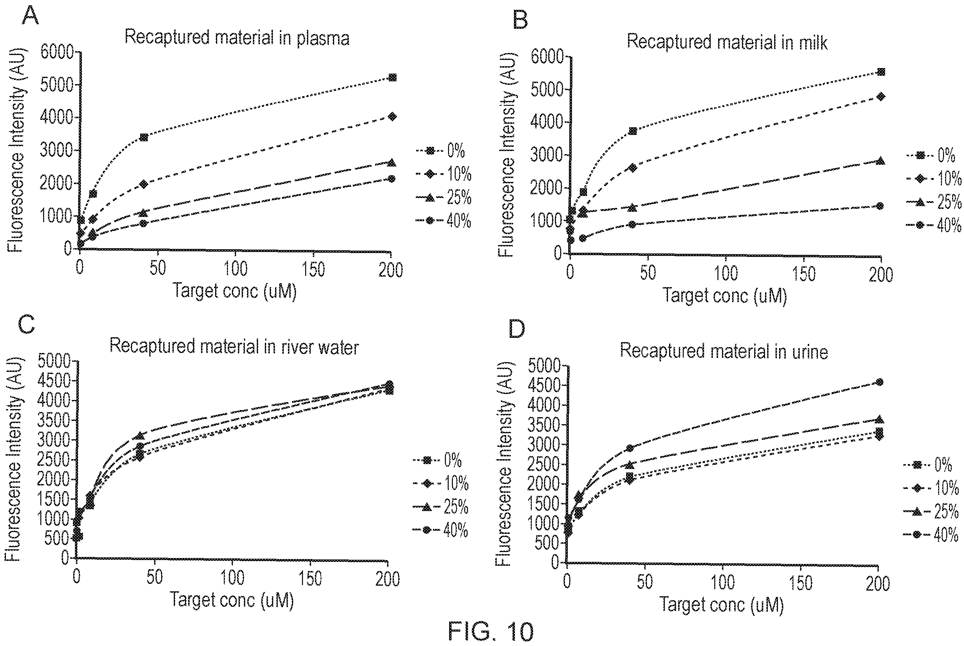

Figure 10 A to D illustrates measured fluorescence signals representing the amount of fluorescent aptamer recaptured by the immobilised 'capture oligonucleotide', (forREV) at 4 different concentrations of test substrates/matrices (human plasma, milk, river water and mock urine, at concentrations of 0%, 10%, 25%, 40% v/v, respectively). Graphs show raw data of fluorescence measurements; and

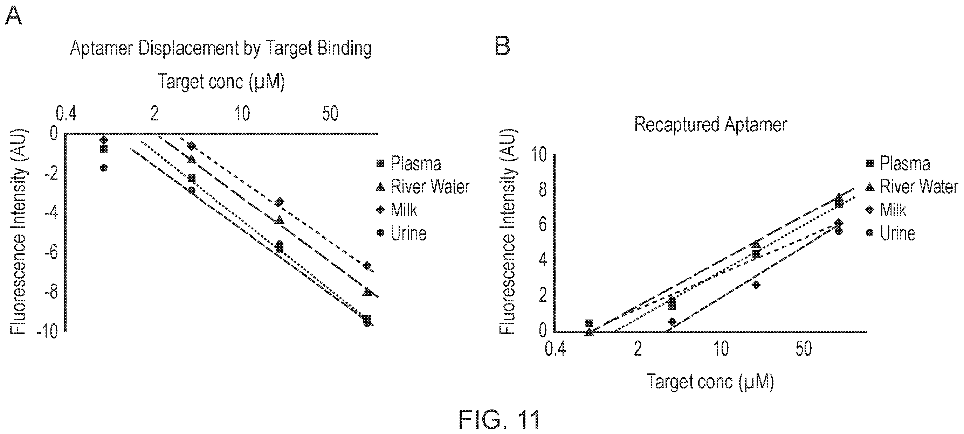

Figure 11 A and B show the results of combined displacement and recapture assays in a single assay. Aptamers were displaced from the first plate (A) and then recaptured on the second plate (B) pre-immobilised with biotinylated 'capture oligo'. The same aptamer and target were tested in a variety of common matrices (plasma, river water, milk and urine) demonstrating the versatility of this platform.

### Detailed Description

Further features of certain embodiments of the present invention are described below.

The practice of embodiments of the present invention will employ, unless otherwise indicated, conventional techniques of molecular biology, microbiology, recombinant DNA technology and immunology, which are within the skill of those working in the art.

Most general molecular biology, microbiology recombinant DNA technology and immunological techniques can be found in Sambrook et al, Molecular Cloning, A Laboratory Manual (2001) Cold Harbor-Laboratory Press, Cold Spring Harbor, N.Y. or Ausubel et al., Current protocols in molecular biology (1990) John Wiley and Sons, N.Y. Unless defined otherwise, all technical and scientific terms used herein have the same meaning as commonly understood by one of ordinary skill in the art to which this disclosure belongs. For example, the Concise Dictionary of Biomedicine and Molecular Biology, Juo, Pei-Show, 2nd ed., 2002, CRC Press; The Dictionary of Cell and Molecular Biology, 3rd ed., Academic Press; and the Oxford University Press, provide a person skilled in the art with a general dictionary of many of the terms used in this disclosure.

Units, prefixes and symbols are denoted in their Système International de Unitese (SI) accepted form. Numeric ranges are inclusive of the numbers defining the range. Unless otherwise indicated, amino acid sequences are written left to right in amino to carboxy orientation and nucleic acid sequences are written left to right in 5' to 3' orientation.

In the following, the invention will be explained in more detail by means of non-limiting examples of specific embodiments. In the example experiments, standard reagents and buffers free from contamination are used.

In a first aspect of the present invention there is provided an apparatus for detecting the presence, absence or level of a target molecule in a sample, the apparatus comprising:
a) a first region comprising:
   i) a support; and
   ii) an immobilisation oligonucleotide; and
   iii) a nucleic acid molecule which has a binding affinity to a target molecule, wherein the nucleic acid molecule is configured to form a complex with the target molecule,
   wherein the immobilisation oligonucleotide is attached directly or indirectly to the support, and further wherein the immobilisation oligonucleotide comprises a nucleic acid sequence which is at least partially complementary to a nucleic acid sequence of the nucleic acid molecule and wherein the nucleic acid molecule is capable of hybridizing to the immobilisation oligonucleotide and further wherein the nucleic acid molecule is capable of being displaced from the complex with the immobilisation oligonucleotide if the target molecule is present in the sample; the apparatus further comprising:
b) a further region comprising:
   i) a support; and
   ii) a capture oligonucleotide attached directly or indirectly to the support, wherein the capture oligonucleotide comprises a nucleic acid sequence which is at least partially complementary to a nucleic acid sequence of the nucleic acid molecule, wherein the capture oligonucleotide is configured to hybridize to the nucleic acid sequence of the nucleic acid molecule when in complex with the target molecule to capture the nucleic acid-molecule-target molecule complex thereby enabling the presence or level of the target molecule to be detected.

In a further aspect of the present invention there is provided a method of detecting the presence, absence or amount of a target molecule in a sample, the method comprising:
a) interacting a sample with a complex comprising:
   i) an immobilisation oligonucleotide; and
   ii) a nucleic acid molecule, wherein the immobilisation oligonucleotide is attached directly or indirectly to a support, the immobilisation oligonucleotide comprising a nucleic acid sequence which is at least partially complementary to a nucleic acid sequence of the nucleic acid molecule and wherein the nucleic acid molecule is capable of hybridizing to a portion of the immobilisation oligonucleotide;
   wherein the nucleic acid molecule has a binding affinity to a target molecule, and further wherein the nucleic acid molecule is configured to form a complex with the target molecule;
b) if the target molecule is present in the sample, disassociating the nucleic acid molecule from the complex with the immobilisation oligonucleotide to form a target molecule-nucleic acid molecule complex; and
c) providing a capture oligonucleotide which comprises a nucleic acid sequence which is at least partially complementary to a nucleic acid sequence of the nucleic acid molecule, wherein the nucleic acid molecule is capable of hybridizing to a portion of the capture oligonucleotide; and
d) detecting the presence or absence of the target molecule.

In certain embodiments, the method comprises contacting the hybridized nucleic acid molecule with a target molecule. In certain embodiments, the method comprises contacting the nucleic acid molecule and the immobilisation oligonucleotide with a sample comprising a target molecule.

When the nucleic acid molecule is contacted with a target molecule, a nucleic acid molecule - target complex is formed by binding the nucleic acid molecule to the target molecule. The binding event results in destabilisation and disruption of the hybridization described above. Consequently, the nucleic acid molecule is displaced from the immobilisation oligonucleotide.

In certain embodiments, the nucleic acid molecule is immobilised on a surface via hybridization with the immobilisation oligonucleotide. Binding of the target molecule causes a conformational change in the nucleic acid molecule which results in its displacement from the immobilisation oligonucleotide and, as a result, displacement from the surface.

Embodiments of the present invention relate to apparatus and methods for detecting the presence and/or absence of a target molecule in a sample using a nucleic acid molecule. As used herein, "nucleic acid molecule" and "aptamer," are used interchangeably to refer to a non-naturally occurring nucleic acid molecule that has a desirable action on a target molecule. For nucleic acid aptamers, for example, a distinction is made between DNA aptamers formed from single-stranded DNA (ssDNA) and RNA aptamers formed from single-stranded RNA (ssRNA). Aptamers can show high binding property to the target molecule, and the affinity thereof is often high compared to antibodies having a similar function. Aptamers are characterised by the formation of a specific three-dimensional structure that depends on the nucleic acid sequence. The three-dimensional structure of an aptamer arises due to Watson and Crick intramolecular base pairing, Hoogsteen base pairing (quadruplex), wobble pair formation or other non-canonical base interactions. This structure enables aptamers, analogous to antigen-antibody binding, to bind target structures accurately. A particular nucleic acid sequence of an aptamer may, under defined conditions, have a three-dimensional structure that is specific to a defined target structure.

The nucleic acid aptamers described herein may comprise natural or non-natural nucleotides and/or or base derivatives (or combinations thereof). In certain embodiments, the nucleic acid molecule comprises one or more modifications such that it comprises a chemical structure other than deoxyribose, ribose, phosphate, adenine (A), guanine (G), cytosine (C), thymine (T), or uracil (U). The nucleic acid molecule may be modified at the nucleobase, at the pentose or at the phosphate backbone.

In certain embodiments, the nucleic acid molecule comprises one or more modified nucleotides. Exemplary modifications include for example nucleotides comprising an alkylation, arylation or acetylation, alkoxylation, halogenation, amino group, or another functional group. Examples of modified nucleotides include 2'-fluoro ribonucleotides, 2'-NH 2 -, 2'-OCH 3 - and 2'-O-methoxyethyl ribonucleotides, which are used for RNA aptamers.

The nucleic acid molecule may be wholly or partly phosphorothioate or DNA, phosphorodithioate or DNA, phosphoroselenoate or DNA, phosphorodiselenoate or DNA, locked nucleic acid (LNA), peptide nucleic acid (PNA), N3'-P5 'phosphoramidate RNA / DNA, cyclohexene nucleic acid (CeNA), tricyclo DNA (tcDNA) or spiegelmer, or the phosphoramidate morpholine (PMO) components (see also Chan et al., Clinical and Experimental Pharmacology and Physiology (2006) 33, 533-540).

Some of the modifications allow nucleic acid molecules to be stabilized against nucleic acid-cleaving enzymes. In the stabilization of the aptamers, a distinction can generally be made between the subsequent modification of the aptamers and the selection with already modified RNA / DNA. The stabilization does not affect the affinity of the modified RNA / DNA aptamers, but prevents the rapid decomposition of the aptamers in an organism or biological solutions by RNases / DNases. An aptamer is referred to as stabilized in the context of the present invention if the half-life in biological sera is greater than one minute, preferably greater than one hour, more preferably greater than one day. The aptamers may also be modified with reporter molecules which, in addition to the detection of the labelled aptamers, may also contribute to increasing the stability.

A desirable action includes, for example, binding of the target molecule, catalytically changing the target molecule, reacting with the target molecule in a way that modifies or alters the target or the functional activity of the target molecule, covalently attaching to the target molecule and facilitating the reaction between the target molecule and another molecule. In the context of embodiments of the present invention, a desirable action is the binding of a target molecule with a high affinity.

In an embodiment, the action is specific binding affinity for a target molecule, wherein the target molecule is a three dimensional chemical structure other than a polynucleotide that binds to the nucleic acid ligand through a mechanism which is independent of Watson/Crick base pairing or triple helix formation, wherein the aptamer is not a nucleic acid having the known physiological function of being bound by the target molecule.

Aptamers to a target molecule may be selected using known processes. For example, an aptamer can be prepared by utilising the SELEX method and an improved method thereof (e.g., Ellington & Szostak, (1990) Nature, 346, 818-822; Tuerk & Gold, (1990) Science, 249, 505-510). In the SELEX method, by setting strict selection conditions by increasing the number of rounds or using a competing substance, an aptamer exhibiting a stronger binding potential for the target molecule is concentrated and selected. Hence, by adjusting the number of rounds of SELEX and/or changing the competitive condition, aptamers with different binding forces, aptamers with different binding modes, and aptamers with the same binding force or binding mode but different base sequences can be obtained in some cases. The SELEX method comprises a process of amplification by PCR; by causing a mutation by using manganese ions and the like in the process, it is possible to perform SELEX with higher diversity.

The variability of a library is for example in the range of about 10¹⁵ different molecules. Starting from the single-stranded DNA or RNA nucleic acid molecule library, the nucleic acid molecules, which bind best to the target, are enriched cycle by cycle via various selection and amplification steps. Each aptamer selection cycle comprises essentially the following substeps:
a) binding of nucleic acid molecule library to target;
b) separating target-bound from unbound nucleic acid molecules;
c) recovery of target-binding nucleic acid molecules;
d) amplification of recovered nucleic acid molecules (e.g. PCR for DNA molecules, reverse transcription PCR for RNA molecules); and
e) preparation of relevant single stranded nucleic acids from the amplified product (e.g. ssDNA purification, in vitro RNA transcription).

After each cycle, the selected and enriched nucleic acid molecule pool is used as the starting material for a next cycle. Typically, 8 to 12 cycles are run through although this number varies depending on the target type, method and efficiency of selection.

In certain embodiments, the method comprises analysing the nucleic acid sequence of an aptamer which has been identified as binding to the target molecule with high binding affinity. After analysing the sequence, the aptamers (including variants, mutants, fragments and derivatives thereof) can be prepared by conventional techniques of chemical DNA and RNA synthesis, which are known to the person skilled in the art. Furthermore, the binding properties of individual aptamers to the target molecule can be investigated.

In certain embodiments, the at least one randomised region is flanked at the 5 'and 3' ends by primer regions. The primer regions serve as primer binding sites for PCR amplification of the library and selected aptamers.

In certain embodiments, nucleic acid molecules which are utilised in the apparatus and methods are selected from a "universal aptamer selection library" that is designed as such that any aptamers which come from this selection process, need little to no adaptation to convert into any of the listed assay formats. In certain embodiments, the "universal aptamer selection library" consists of following functional parts: 5' primer sequence, at least one hybridization/capture region (complementary to a "immobilisation oligonucleotide"), at least one randomised region and a 3' primer sequence. Once selected, the nucleic acid molecule may be further modified e.g. to remove one or both primer sequences before being used. In certain embodiments and as described above, the fixed primer sequences may be used as the capture sequence. Thus in certain embodiments, the capture oligonucleotide comprises a nucleic acid sequence which is complementary to one of the primer sequences in the nucleic acid molecule. This permits the development of methods and apparatus which utilise a capture oligonucleotide which comprises the same nucleic acid sequence regardless of the target molecule to be detected.

Aptamers are altered easily since they permit chemical synthesis. For aptamers, by predicting the secondary structure using the MFOLD program, or by predicting the steric structure by X-ray analysis or NMR analysis, it is possible to predict to some extent which nucleotide can be substituted or deleted, where to insert a new nucleotide and the like. A predicted aptamer with the new sequence can easily be chemically synthesized, and it can be determined whether or not the aptamer retains the activity using an existing assay system.

Aptamers for use in the apparatus and methods of certain embodiments of the present invention can be synthesized by methods known per se in the art. One of the synthesis methods is a method using an RNA polymerase. The object RNA can be obtained by chemically synthesizing a DNA having the object sequence and a promoter sequence of RNA polymerase, followed by in vitro transcription using same as a template and according to an already-known method.

It can be synthesized using DNA polymerase. DNA having an object sequence is chemically synthesized and, using same as a template, amplification is performed by a known method of polymerase chain reaction (PCR). This is converted to a single strand by an already-known method of polyacrylamide electrophoresis or enzyme treatment method. When a modified aptamer is synthesized, the efficiency of elongation reaction can be increased by using a polymerase introduced with a mutation into a specific site. The thus-obtained aptamer can be purified easily by a known method.

The aptamers can be synthesized in a large amount by a chemical synthesis method such as amidite method, phosphoramidite method and the like. The synthesis method is a well-known method, and as described in Nucleic Acid (Vol. 2 )1] Synthesis and Analysis of Nucleic Acid (Editor: Yukio Sugiura, Hirokawa Publishing Company) and the like. In fact, a synthesizer such as OligoPilot100, OligoProcess and the like manufactured by GE Healthcare Bioscience is used. Purification is performed by a method known per se such as chromatography and the like.

### Target Molecule

The apparatus and methods described herein are for use to detect the presence or absence and/or quantify the amount of a target molecule in a sample. The term "target molecule" as used herein denotes a molecule which may be found in a tested sample and which is capable of binding to a nucleic acid molecule described herein.

In accordance with some embodiments, the target molecule is an organic molecule. In some embodiments, the target molecule is a soluble antigen, a cell-surface antigen, or an antigen associated with a micelle, a liposome or a particle. In one embodiment, the target molecule is an antigen.

In some embodiments, the target molecule may be a protein, a polypeptide, a peptide, a ganglioside, a lipid, a phospholipid, a carbohydrate, a small molecule or a nucleic acid molecule.

In certain embodiments, a soluble antigen may be a protein, a peptide, an enzyme, a cytokine, a soluble cancer marker, an inflammation-associated marker, a hormone and/or a soluble molecule derived from a virus, bacteria or a fungus for example, a toxin or an allergen.

In some embodiments, the antigen is a micro-organism associated antigen. As used herein, the term "micro-organism associated antigen" is to be understood as a protein or fragment thereof encoded by the viral, bacterial or fungal genome.

In certain embodiments, the antigen is a cancer (or tumour) marker. In general, a tumour marker may be found in the body fluids such as in blood or urine, or in body tissues. Tumour markers may be expressed or over expressed in cancer and are generally indicative of a particular disease process.

Non-limiting examples of a cell surface antigen in accordance with the invention are a receptor, a cell surface marker, a micro-organism associated antigen, or a receptor ligand.

In certain embodiments, the target molecule is a small molecule. In certain embodiments, the small molecule is a therapeutic agent for example a chemotherapeutic agent which is for use in the treatment of cancer.

### Sample

The target molecule may be comprised in a sample. The sample may comprise whole blood, leukocytes, peripheral blood mononuclear cells, plasma, serum, sputum, breath, urine, semen, saliva, meningial fluid, amniotic fluid, glandular fluid, lymph fluid, nipple aspirate, bronchial aspirate, synovial fluid, joint aspirate, cells, a cellular extract, stool, tissue, a tissue biopsy, and cerebrospinal fluid.

The sample may comprise blood, serum, interstitial fluid, spinal fluid, cerebral fluid, tissue exudates, macerated tissue samples, ceil solutions, intracellular compartments, groundwater, or other biological and environmental samples. Samples may be unaltered or may be pretreated prior to analysis, for example being filtered, diluted, concentrated, buffered, or otherwise treated.

A sample is aptly a material provided or sampled which is believed to contain one or more target molecules of interest e.g. a small molecular analyte(s) of interest) and which should be checked for the presence of the target.

The sample may be for example a clinical sample, a food sample, a water sample or a sample of other environmental sampled material. In certain embodiments, the sample comprises a target molecule and a buffer solution. In certain embodiments, the sample is pre-treated, such as by mixing, addition of enzymes or markers, or purified.

In certain embodiments, the sample may further be any biological material isolated from individuals, for example, biological tissues and fluids, which include, but are not limited to: Count blood, skin, plasma, serum, lymph, urine, cerebrospinal fluid, tears, swabs, tissue samples, organs and tumours. In certain embodiments, components of cell cultures are also included in samples.

Other exemplary target molecules and samples are described herein.

### Immobilisation oligonucleotide

Aptly, the immobilisation oligonucleotide comprises a nucleic acid sequence which is configured to hybridise to a nucleic acid sequence of the nucleic acid molecule over at least a portion of its length.

In certain embodiments, the immobilisation region of the nucleic acid molecule hybridizes over at least a portion thereof to a region of the immobilisation oligonucleotide. The terms "hybridizes" and "hybridization" as used herein mean to form an interaction based on Watson-crick base pairing between a fixed region within the aptamer library and a complimentary sequence within the 'immobilisation oligonucleotide', under conventional hybridization conditions, preferably under stringent conditions, as described, for example, in Sambrook et al., Molecular Cloning, A Laboratory Manual, 3. Ed. (2001) Cold Spring Harbor Laboratory Press, Cold Spring Harbor, NY.

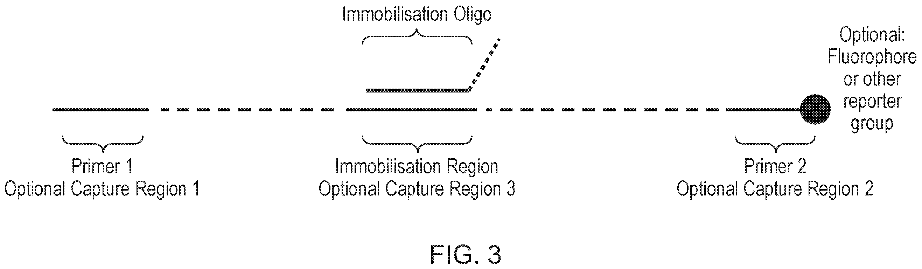

The immobilisation oligonucleotide or portion thereof is configured to form a double-stranded duplex structure with the immobilisation region or a portion thereof of the nucleic acid molecule. In certain embodiments, the immobilisation oligonucleotide is between approximately 10 nucleotides and about 20 nucleotides in length, e.g. 10, 11, 12, 13, 14, 15, 16, 1, 18, 19 or 20 nucleotides in length. The immobilisation oligonucleotide may comprise a nucleic acid sequence which is complementary to a fixed sequence of the nucleic acid molecule. Thus, in some embodiments, the immobilisation oligonucleotide may be a "universal" oligonucleotide in that it may be configured to hybridize to a sequence which can be included in a plurality of nucleic acid molecules. A schematic representation is provided in Figure 3.

In certain embodiments, the nucleic acid molecule and the immobilisation oligonucleotide are incubated under conditions sufficient for the nucleic acid molecule or portion thereof to hybridize to the immobilisation oligonucleotide.

A person skilled in the art will understand that the conditions required for hybridization to occur will vary between reactions. In certain embodiments, the method comprises heating a mixture of the nucleic acid molecule and the immobilisation oligonucleotide e.g. to at least about 80°C, e.g. 85°C, 86°C, 87°C, 88°C, 89°C, or 90°C for at least about 1 minute e.g. 2 minutes, 3 minutes, 4 minutes, 5 minutes or more. The method may further comprise cooling the nucleic acid molecule and the immobilisation oligonucleotide to a temperature lower than about 10 °C, e.g. 9°C, 8°C, 7°C, 6°C, 5°C, 4°C or lower.

In certain embodiments, the hybridization is carried out prior to immobilisation of the immobilisation oligonucleotide to the support. In an alternative embodiment, the immobilisation oligonucleotide is attached to the support prior to hybridization of the nucleic acid molecule to the immobilization oligonucleotide.

The immobilisation oligonucleotide is attached to the support of the first region. The attachment may be directly or indirectly e.g. via a linker or other attachment moiety.

In certain embodiments, the immobilisation oligonucleotide comprises a linker portion. The linker portion may be selected from biotin, thiol, and amine. The immobilisation oligonucleotide may further comprise a spacer molecule e.g. a spacer molecule selected from a polynucleotide molecule, C6 spacer molecule, a C12 spacer molecule, another length C spacer molecule, a hexaethylene glycol molecule, a hexanediol, and/or a polyethylenglycol.

The linker may be for example a biotin linker. In certain embodiments, the aptamer may be conjugated to avidin. Further details on linkers are found herein. In certain embodiments, the linker and/ or the spacer molecule is a photocleavable molecule e.g. 5-bromouracil and/or 5-iodouracil.

In certain embodiments, the immobilisation oligonucleotide may be modified for attachment to the support surface. For example, the immobilisation oligonucleotide may be attached via a silane linkage. Alternatively or in addition, the immobilisation oligonucleotide may be succinylated (e.g. to attach the immobilisation oligonucleotide to aminophenyl or aminopropyl-derivatized glass). Aptly, the support is aminophenyl or aminopropyl-derivatized. In certain embodiments, the immobilisation oligonucleotide comprises a NH₂ modification (e.g. to attach the oligonucleotide to epoxy silane or isothiocyanate coated glass). Aptly, the support surface is coated with an epoxy silane or isothiocyanate. In certain embodiments, the immobilisation oligonucleotide is hydrazide-modified in order to attach to an aldehyde or epoxide molecule.

### Support

The apparatus of certain embodiments comprises a first region which comprises a support. The support may comprise a solid state support such as a membrane or a bead. The support may be a two dimensional support e.g. a microplate or a three dimensional support e.g. a bead. Thus, in certain embodiments the support may comprise at least one magnetic bead. In alternative embodiments, the support may comprise at least one nanoparticle e.g. gold nanoparticles or the like. In yet further embodiments, the support of the first region comprises a microtiter or other assay plate, a strip, a membrane, a film, a gel, a chip, a microparticle, a nanofiber, a nanotube, a micelle, a micropore, a nanopore, and a biosensor surface. In certain embodiments, the biosensor surface may be a probe tip surface, a biosensor flow-channel or similar. In certain embodiments, the further region comprises a support. The supports of the first and further regions may be a continuous element. Alternatively, the supports of the first and further regions may be separate elements.

Particularly suitable materials from which a support can be made include for example inorganic polymers, organic polymers, glasses, organic and inorganic crystals, minerals, oxides, ceramics, metals, especially precious metals, carbon and semiconductors. A particularly suitable organic polymer is a polymer based on polystyrene. Biopolymers, such as cellulose, dextran, agar, agarose and Sephadex, which may be functionalized, in particular as nitrocellulose or cyanogen bromide Sephadex, can be used as polymers which provide a solid support.

In certain embodiments, the immobilisation oligonucleotide may be attached, directly or indirectly, to a magnetic bead, which may be e.g. carboxy-terminated, avidin-modified or epoxy-activated or otherwise modified with a compatible reactive group. The examples of polymers are not limiting and other molecules are conceivable.

Immobilisation of oligonucleotides to a support e.g. a solid phase support can be accomplished in a variety of ways and in any manner known to those skilled in the art for immobilising DNA or RNA on solids. The immobilisation of aptamers on nanoparticles is e.g. as described in WO2005/13817. For example, a solid phase of paper or a porous material may be wetted with the liquid phase aptamer, and the liquid phase subsequently volatilized leaving the aptamer in the paper or porous material.

In certain embodiments, the support of the first and/or further region comprises a membrane, e.g. a nitrocellulose, a polyethylene (PE), a polytetrafluoroethylene (PTFE), a polypropylene (PP), a cellulose acetate (CA), a polyacrylonitrile (PAN), a polyimide (PI), a polysulfone (PS), a polyethersulfone (PES) membrane or an inorganic membrane comprising aluminum oxide (Al2O3), silicon oxide (SiO2) and/or zirconium oxide (ZrO2).

Particularly suitable materials from which a support can be made include for example inorganic polymers, organic polymers, glasses, organic and inorganic crystals, minerals, oxides, ceramics, metals, especially precious metals, carbon and semiconductors. A particularly suitable organic polymer is a polymer based on polystyrene. Biopolymers, such as cellulose, dextran, agar, agarose and Sephadex, which may be functionalized, in particular as nitrocellulose or cyanogen bromide Sephadex, can be used as polymers which provide a solid support.

### Further region

The apparatus also comprises a further region which comprises a support. The support of the further region may be the same or may be different to the support of the first region. The further region may be comprised in a different vessel or apparatus to the first region.

The further region also includes a capture oligonucleotide attached directly or indirectly to the further support. The capture oligonucleotide may comprise the same sequence as the immobilisation oligo nucleotide. Alternatively, the capture oligonucleotide may comprise a different sequence to the immobilisation oligonucleotide. The further region may comprise a plurality of capture oligonucleotides which bind to the nucleic acid molecule at a different sequence thereof.

The capture oligonucleotide or portion thereof is configured to form a double-stranded duplex structure with a sequence of the nucleic acid molecule. In certain embodiments, the capture oligonucleotide is between approximately 10 nucleotides and about 20 nucleotides in length, e.g. 10, 11, 12, 13, 14, 15, 16, 17, 18, 19 or 20 nucleotides in length. The capture oligonucleotide may comprise a nucleic acid sequence which is complementary to a fixed sequence of the nucleic acid molecule. Thus, in some embodiments, the immobilisation oligonucleotide may be a "universal" oligonucleotide in that it may be configured to hybridize to a sequence which can be included in a plurality of nucleic acid molecules.

### Detectable Labels

In certain embodiments, the nucleic acid molecule comprises a detectable label. In certain embodiments, the capture oligonucleotide comprises a detectable molecule. In certain embodiments the detectable label is a fluorescent moiety, e.g. a fluorescent/quencher compound. Fluorescent/quencher compounds are known in the art, see for example Mary Katherine Johansson, Methods in Molecular Biol. 335:Fluorescent Energy Transfer Nucleic Acid Probes: Designs and Protocols, 2006, Didenko, ed., Humana Press, Totowa, NJ, and Marras et al., 2002, Nucl. Acids Res. 30, el22.

Further, moieties that result in an increase in detectable signal when in proximity of each other may be used as alternative labels in the apparatus and methods described herein, for example, as a result of fluorescence resonance energy transfer ("FRET"); suitable pairs include but are not limited to fluoroscein and tetramethylrhodamine; rhodamine 6G and malachite green, and FITC and thiosemicarbazole, to name a few.

In certain embodiments, the detectable label is selected from a fluorophore, a nanoparticle, a quantum dot, an enzyme, a radioactive isotope, a pre-defined sequence portion, a biotin, a desthiobiotin, a thiol group, an amine group, an azide, an aminoallyl group, a digoxigenin, an antibody, a catalyst, a colloidal metallic particle, a colloidal non-metallic particle, an organic polymer, a latex particle, a nanofiber, a nanotube, a dendrimer, a protein, and a liposome.

In certain embodiments, the detectable label is an enzyme, wherein optionally the enzyme is selected from horseradish peroxidase, alkaline phosphatase, urease and β-galactosidase.

In certain embodiments, the nature of the detection means will be dependent on the detectable label used. For example, in certain embodiments, the label may be detectable by virtue of its colour e.g. gold nanoparticles. A colour can be detected quantitatively by an optical reader or camera e.g. a camera with imaging software. In certain embodiments, if the detectable label is a fluorescent label e.g. a quantum dot, the detection means may comprise a fluorescent strip reader which is configured to record fluorescence intensity for example.

In embodiments in which the detectable label is an enzyme label the detection means may be an electrochemical detector. The detection label may further comprise enzymes such as horseradish peroxidase (HRP), Alkaline phosphatase (APP) or similar, to catalytically turnover a substrate to give an amplified signal.

In certain embodiments, the method comprises detecting the formation of the nucleic acid molecule-target molecule complex. As discussed herein, when the aptamer is contacted with the target, an aptamer-target complex is formed by binding the aptamer to the target. The binding or binding event can be detected, for example, visually, optically, photonically, electronically, acoustically, opto-acoustically, by mass, electrochemically, electro-optically, spectrometrically, enzymatically or otherwise chemically, biochemically or physically.

In certain embodiments, the nucleic acid molecule may comprise a detection molecule e.g. a label. Exemplary labels are visual, optical, photonic, electronic, acoustic, opto-acoustic, mass, electrochemical, electro-optical, spectrometric, enzymatic, or otherwise physically, chemically or biochemically detectable. In one embodiment of the method, the label is detected by luminescence, UV / VIS spectroscopy, enzymatically, electrochemically or radioactively. Luminescence refers to the emission of light. In the method according to certain embodiments of the invention, for example, photoluminescence, chemiluminescence and bioluminescence are used for detection of the label. In photoluminescence or fluorescence, excitation occurs by absorption of photons. Exemplary fluorophores include, without limitation, bisbenzimidazole, fluorescein, acridine orange, Cy5, Cy3 or propidium iodide, which can be covalently coupled to aptamers, tetramethyl-6-carboxyhodamine (TAMRA), Texas Red (TR), rhodamine, Alexa Fluor dyes (et al. Fluorescent dyes of different wavelengths from different companies).

Other tags are catalysts, colloidal metallic particles, e.g. as gold nanoparticles, colloidal non-metallic particles, quantum dots, organic polymers, latex particles, nanofibers, in particular carbon, nanotubes, in particular carbon (carbon nanotubes), dendrimers, proteins or liposomes with signal-generating substances. Colloidal particles can be detected calori metrically.

In certain embodiments, the detectable molecule is an enzyme. In certain embodiments, the enzyme may convert substrates to coloured products, e.g. peroxidase, Green Fluorescent Protein (GFP), luciferase, β-galactosidase or alkaline phosphatase. For example, the colourless substrate X-gal is converted by the activity of β-galactosidase to a blue product whose colour is visually detected.

An above-mentioned enzymatic marker and detection system uses alkaline phosphatase. With alkaline phosphatase (APP), various detections are possible, as exemplified below: electrochemical detection: substrate phenyl phosphate, enzymatic reaction of APP forms phenol, is attached to phenol sensor (e.g. electrochemically detected measurement of the colour reaction: substrate p-nitrophenyl phosphate, enzymatic reaction of APP forms p-nitrophenol, is coloured yellow fluorescence detection: substrate 4-methylumbelliferonyl phosphate: enzymatic reaction of APP forms methylumbelliferonyl residue, which after excitation fluorescence released for chemiluminescence detection:
Furthermore, APP selectively converts 5-bromo-4-chloro-3-indolyl phosphate (BCIP or X-phosphate) and nitroblue tetrazolium salt (NBT). The dyes precipitate in the immediate vicinity of the AP molecules and colour the surroundings of the bound compounds dark purple.

The peroxidase catalyses for example, the oxidation of ABTS (2,2'-azino-bis- [3-ethylbenzthiazoline-6-sulfonic acid]) in the presence of H₂O₂. Due to the enzyme stability and a large number of possible substrates, horseradish peroxidase is preferred. Other enzyme markers that catalyse the generation of detectable products are chloramphenicol acetyltransferase (CAT) and glutathione-S-transferase (GST).

In certain embodiments, the detectable molecule is a radioactive isotope. The detection can also be carried out by means of radioactive isotopes with which the aptamer is labelled, preferably 3H, 14C, 32P, 33P, 35S or 1251, more preferably 32P, 33P or 1251. In the scintillation counting, the radioactive radiation emitted by the radioactively labelled aptamer-target complex is measured indirectly. A scintillator substance is excited by the radioactive radiation. During the transition to the ground state, the excitation energy is released again as flashes of light, which are amplified and counted by a photomultiplier.

In certain embodiments, the detectable molecule is selected from digoxigenin and biotin. Thus, the aptamers may also be labelled with digoxigenin or biotin, which are bound for example by antibodies or streptavidin, which may in turn carry a label, such as e.g. an enzyme conjugate. The prior covalent linkage (conjugation) of an aptamer with an enzyme can be accomplished in several known ways. Detection of aptamer binding may also be radioactive in an RIA (radioactive immunoassay) with radioactive isotopes, preferably with 1251, or by fluorescence in a FIA (fluoroimmunoassay) with fluorophores, preferably with fluorescein or FITC.

The apparatus may further comprise a sample introduction region. The sample introduction region may comprise a port and may be provided upstream from the first region.

In certain embodiments, the apparatus may further comprise:
a) one or more competitor molecules; and
b) one or more control molecules.

In a certain embodiment, the competitor molecules are defined amounts of unspecific and/or negative target molecules which enable discrimination with the sample.

In a certain embodiment, the control molecules are defined amounts of target molecules which enable comparison with the sample.

In another embodiment, the control molecule may be a non-functional DNA/RNA sequence (control DNA/RNA sequence) which is complementary to a control Capture molecule that is located in a control zone (described below). Hybridisation of the control DNA/RNA sequence to the control Capture molecule would prove principle functionality of the assay.

### Apparatus

The apparatus according to the first aspect of the present invention may be provided in a number of different formats. In certain embodiments, the apparatus may be a biosensor. Biosensors are found in many different formats. In a further aspect, the invention relates to a biosensor comprising an aptamer according to the invention.

In certain embodiments, the biosensor comprises the following elements: a first region comprising the nucleic acid molecule and a transducer which converts the binding event between a nucleic acid molecule and a target molecule into an electrically quantifiable signal. The first region may be comprised in a vessel or a probe or the like.

In addition, the apparatus may further comprise other elements, such as a signal processing device, an output electronics, a display device, a data processing device, a data memory device and interfaces to other devices.

From a sample which is brought into contact with the biosensor, for example a complex mixture containing the target molecule, the target molecule can be identified via the specific binding of the nucleic acid molecule.

The sensitivity of the sensor may be influenced by the transducer used. The nucleic acid molecule-target molecule complex binding to the capture oligonucleotide may be converted by the transducer into an electronically usable signal. The transducer converts the signal from binding event, which is proportional to the concentration of the target molecule in the sample, into an electrically quantifiable measurement signal. Signalling occurs due to the molecular interaction between the nucleic acid molecule.

With a biosensor according to certain embodiments of the present invention, qualitative, quantitative and / or semi-quantitative analytical information can be obtained. The apparatus according to certain embodiments may be particularly suitable for use in food, water and environmental analysis, and water treatment, especially of drinking water and wastewater, in diagnostics and for use in hospitals and care facilities.

In certain embodiments the first and/or further region may comprise the transducer surface, and the release and re-capture events described herein may be detected by the transducer.

The recapture of the target-molecule-nucleic acid molecule complex can be measured, for example, via optical, microgravimetric, thermal or acoustic transducers, e.g. via optical or microgravimetric transducers. The measurement in optical transducers can be based on principles of photometry, whereby, for example, colour or luminescence intensity changes are detected. Optical methods include the measurement of fluorescence, phosphorescence, bioluminescence and chemiluminescence, infrared transitions and light scattering. The optical methods also include the measurement of layer thickness changes when target is bound to aptamer. The layer thickness change can, e.g. by surface plasmon resonance (SPR), biolayer interferometry (BLI) or reflectometric interference spectroscopy (RlfS) are performed. Furthermore, the interference on thin layers (reflektrometric interference spectroscopy) and the change of the evanescent field can be measured.

Acoustic transducers use the frequency changes of a piezoelectric quartz crystal, which detects highly sensitive mass changes that occur when target binds to aptamer. The quartz crystal used is placed in an oscillating electric field and the resonant frequency of the crystal is measured. A mass change on the surface of the quartz crystal, e.g. by reaction of the analyte with the previously immobilized on the crystal surface receptor causes a change in the resonant frequency, which can be quantified.

Electrochemical transducers may, e.g. measuring the change in the concentration of redox-active markers on the electrode surface, or changing the concentration of redox-active substrates or products, the e.g. are consumed or formed in the enzymatic reaction of an enzyme marker. Thermal transducers measure the heat of the aptamer-target binding reaction.

In certain embodiments, the apparatus is a BLI (Biolayer Interferometry) apparatus or similar apparatus. In certain embodiments, the first region is comprised on a Biosensor probe. The further region containing the Capture oligonucleotide is comprised on a second Biosensor probe. The first probe is incubated with the sample. If the target molecule is present in the sample, the nucleic acid molecule is displaced from the complex with the immobilisation oligonucleotide, to form a target molecule-nucleic acid molecule complex. As a consequence, the nucleic acid sequence is displaced from the probe and present in the sample. The displacement of the nucleic acid molecule gives a quantifiable reduction in signal and can be measured by BLI. The second probe is then incubated with the sample containing the nucleic acid molecule-target complex. The re-capturing of the nucleic acid molecule or the nucleic acid molecule-target complex gives a quantifiable association signal and can be measured by BLI.

Depending on the design, qualitative, quantitative and / or semi-quantitative analytical information about the target to be measured can be obtained with the measuring device. The detection means may be for example a portable meter that can be used on-site, such as a pocket-sized lightweight meter.

In certain embodiments, the apparatus comprises a lateral flow device. For example, the biosensor may be a lateral flow device. Lateral flow devices may also be referred to as lateral flow tests, lateral flow assays and lateral flow immunoassays.

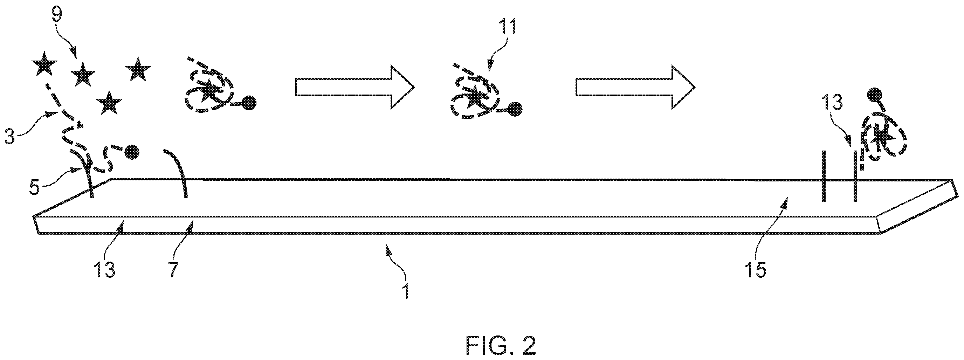

Figure 2 is a schematic representation of a lateral flow device 1. The lateral flow device comprises a first region 13 which comprises a support 7 onto which an immobilisation oligonucleotide 5 is attached. The immobilisation oligonucleotide is configured to hybridise to a nucleic acid molecule e.g. an aptamer 3 over a portion of its sequence.

A sample is introduced to the first region. The sample is aptly a liquid. If the sample comprises a target molecule 9, the aptamer binds to the target molecule and undergoes a conformational change, which results in the aptamer disassociating from the immobilisation oligonucleotide. The target molecule-aptamer complex 11 is then free in solution. Aptly, the lateral flow device comprises a flow path from the first region to a further region 15. The target molecule-aptamer complex flows from the first region to the further region.

The further region 15 aptly comprises a test region which includes a capture oligonucleotide 13. The capture oligonucleotide 13 comprises a nucleic acid sequence which is complementary to a sequence of the aptamer when the aptamer is in complex with the target molecule. The aptamer when in a complex with the target molecule is then recaptured in the test region. The presence of the aptamer-target molecule complex in the test region can then be detected and optionally quantified thus providing a measurement of the presence and optionally amount of target molecule in the sample.

Aptly, the lateral flow device comprises a control region e.g. a control line which is configured to confirm that the test is working. Furthermore, the lateral flow device aptly comprises one or more target test lines.

The lateral flow device may be in the form of a strip. Aptly the support comprises a membrane e.g. a nitrocellulose membrane. The membrane may be formed from non-woven or woven material. The support may further comprise a pad, e.g. an absorbent pad, to which the sample can be applied. Aptly the absorbent pad is configured to wick the sample through the membrane. In certain embodiments, the absorbent pad comprises cellulose fibers.

In certain embodiments, the apparatus may further comprise a backing element which is adjacent to the support.

The lateral flow assay may work as follows:
Selected aptamers are immobilised onto a support e.g. a 'conjugation pad' by hybridisation to the complementary sequence in an immobilisation oligonucleotide.

Binding of the target molecule causes a conformational change in the aptamer which results in disruption of the hybridisation and subsequently its displacement from the immobilisation oligonucleotide. Displaced aptamers in complex with the target molecule are carried along the LFD strip, in the sample matrix. Displaced aptamers are recaptured by hybridisation to a second immobilised complimentary oligonucleotide forming a 'test line' on the LFD. Additional aptamers against other target molecules may also be run in the same sample and captured on other immobilised complimentary oligonucleotide sequences in other 'test lines', giving rise to a multiplexed LFD assay.

A liquid sample suspected of containing a target for the aptamer identified using the method of certain embodiments of the present invention is applied to the first region at one location, or the strip is wetted with the sample at one site. This place may be the so-called 'sample application pad / zone' of the strip. The strip contains a matrix material through which the liquid sample medium and the target molecule suspended or dissolved therein can flow by capillary action from a sample application pad / zone into a detection zone where a detectable signal or the absence of such a signal indicates the presence or absence of the target.

As described herein, a labelled aptamer is used, wherein any label already described above can be used. Preferably, a label is used which leads to a visually detectable signal in the detection zone of the test strip. The presence or absence of target in the sample can be detected by the presence or absence of a labelled aptamer in the detection zone.

In one embodiment, the support comprises a test strip. In certain embodiments, an enzyme-labelled aptamer is used. If the target molecule is present in the sample it forms a complex with the enzyme-labelled aptamer, which flows along the strip to a detection zone containing a substrate for the enzyme label which is capable of producing a coloured reaction in the presence of the enzyme label. In certain embodiments, the strip contains another zone in which target is immobilised so that labelled aptamer which does not unite with the target due to the absence of sufficient target in the sample is detected and thereby prevented from reaching the detection zone.

The support e.g. the test strip can be produced from any material which can be wetted with a sample and into which an aptamer according to certain embodiments can be introduced. Particularly preferred are materials through which a sample and a target contained therein can flow by capillary action. Examples are nitrocellulose, nitrocellulose blends with polyester or cellulose, uncoated paper, porous paper, viscose filament, glass fiber, acrylonitrile copolymer or nylon, as well as all other materials common in lateral flow assays.

The test strip can already be used as such to detect the presence of target in a sample. For use, the strip may be immersed in a sample, in the case of a lateral flow assay, for example, with only one end, which then serves as the first region i.e. a sample application zone.

Certain embodiments of the present invention relate to a lateral flow assay device comprising the test strip described above. In addition to the test strip, the device may comprise, for example, a housing in which the test strip is embedded and which has a preferably closable opening to the sample application zone of the test strip. In certain embodiments the apparatus comprises a control zone (a zone which is separate to the detection zone, which proves general functionality of the test strip).

In a certain embodiment, the control zone may comprise of a control Capture sequence which is complementary to a non-target binding control DNA/RNA sequence. Hybridisation of the control DNA/RNA sequence to the control Capture molecule would give a positive signal which is distinct from the test signal and proves principle functionality of the assay.

In certain embodiments, the apparatus comprises at least one vessel. The first region may be comprised in a first vessel and the further region may be comprised in a further vessel. In certain embodiments, the apparatus may be suitable for use in an ELONA assay. A schematic representation of an ELONA apparatus is shown in Figure 4. The apparatus 100 may comprise a first region 110 which is comprised in a vessel 120. A further region 120 is comprised in a further vessel 130. A nucleic acid molecule 150 specific to a target molecule 170 is provided immobilized via hybridization to an immobilisation oligonucleotide 160 in the first region. The immobilization oligonucleotide is in certain embodiments immobilized to surface of the vessel 120. A sample which may contain a target molecule is added to the vessel. If the sample contains the target molecule, the target molecule binds to the aptamer resulting in a conformational change which in turn results in displacement of the aptamer from the immobilisation oligonucleotide. This is depicted in Figure 4, upper second image. Following a predetermined period of time sufficient to allow the displacement to occur, the assay sample is transferred to a further vessel 130 which comprises a capture oligonucleotide immobilised on a surface of the well. The capture oligonucleotide comprises a sequence which is complementary to a nucleic acid sequence of the aptamer. A detection molecule 140, for example a streptavidin conjugated enzyme such as HRP is captured on the aptamer (when the aptamer is biotinylated) thus allowing detection with an enzyme substrate (e.g. TMB).

The lower section of Figure 4 illustrates an ELONA assay when the target molecule is not present in the sample. Selected aptamers are immobilised onto the assay well by hybridisation to the complementary sequence in the immobilization oligonucleotide (which is in turn immobilised on a surface of the first vessel). The addition of non-target molecules in the sample has no effect on the aptamer which remain immobilised on the immobilization oligonucleotide. The assay sample is transferred to the further vessel and no aptamers are captured on the capture oligonucleotide. The streptavidin conjugated enzyme (e.g. HRP) is not captured so no signal is measured with enzymes substrates (e.g. TMB).

In another specific embodiment, the solid support and the aptamer form a microarray, or a so-called "DNA or RNA aptamer chip," which may be single-channel or multi-channel. In the microarray, one or more aptamers as well as reference materials for basic signal compensation can be arranged on several array-shaped measuring points. In a multi-channel chip, this arrangement takes place in the individual channels. Thus, the parallel multiple measurement of a sample, or with different aptamers, the parallel measurement of different analytes in a sample is possible.

### EXAMPLES

In the following, the invention will be explained in more detail by means of non-limiting examples of specific embodiments. In the example experiments, standard reagents and buffers free from contamination are used.

### EXAMPLE 1: Biosensor Assay (Aptamer-based assay to detect small molecules using Biolayer Interferometry - BLI)

### Oligonucleotides and Aptamers

The assay was performed using nucleic acid molecules (DNA aptamers) specific for the chemotherapeutic drug Imatinib. Aptamer A was 100 nucleotides in length and comprises a 5'FAM (fluorescein) label. The nucleotide sequence of Aptamer A is set forth below:

5’-
ATCCACGCTCTTTTTCTCCCCCCGCTATGTGAGGCTCGATCGTTCGGTGTGTTTTTAAA
GGGTACAGATCCTGGGCGGGGGGCATTGAGGGTGACATAGG- 3’ (SEQ ID NO: 1)

The underlined sequence relates to first and second primer sites used for amplification of the aptamer but also used for recapture of the displaced aptamer, and the italic sequence relates to the immobilisation region of the aptamer (i.e. nucleic acid sequence of the aptamer capable of binding to at least a portion of immobilisation sequence).

The aptamer was HPLC purified prior to use and was manufactured by IDT, Belgium). For the assay, the aptamer is immobilised on to streptavidin-coated sensor probes via the biotinylated immobilisation oligonucleotide comprising a 5' Biotin label and an internal HEGL spacer ISP18. The immobilisation oligonucleotide was HPLC purified prior to use and also manufactured by IDT, Belgium. The immobilisation oligonucleotide contains a defined region of 12 nucleotides (GATCGAGCCTCA, SEQ ID NO: 2) which is reverse complementary to a region of the aptamer (shown in italics) which enables hybridisation between both molecules. In addition, the immobilisation oligonucleotide carries a 5' biotin bound via a hexaethylene glycol (HEGL) residue, which is responsible for the coupling of the immobilisation oligonucleotide to the streptavidin-modified magnetic beads.

To re-capture aptamer-target complexes, two different capture oligonucleotides were used: the oligonucleotides revFOR and REV. Both capture oligonucleotides contain defined regions of 19 nucleotides which are complementary to the first and last region of the aptamer, respectively, and enable hybridisation between aptamer and capture oligonucleotides. revFOR was 19 nucleotides in length and comprises a 5' Biotin label. The capture oligonucleotide was HPLC purified prior to use and was manufactured by IDT, Belgium. REV was 19 nucleotides in length and comprises a 3' Biotin label. The capture oligonucleotide was HPLC purified prior to use and was manufactured by IDT, Belgium. The nucleotide sequences of the capture oligonucleotides are shown below:
revFOR - 5'-GGAGAAAAAGAGCGTGGAT-3' (SEQ ID NO:3)
REV - 5'-CCTATGTCACCCTCAATGC-3' (SEQ ID NO:4).

### Buffers, Target

Buffer BB (20 mM Tris-HCl pH 7.6, 100 mM NaCl, 5 mM KCI, 2 mM MgCl₂, 1 mM CaCl₂, 0.01 % Tween 20) served as hybridisation buffer. Buffer B&W (5 mM Tris-HCl pH 7.5, 0.5 mM EDTA, 1 M NaCl, 0.01% Tween 20) was used to immobilise biotinylated oligonucleotides onto streptavidin-coated surfaces. Buffer PBS6 (10 mM Na₂HPO₄/2 mM KH₂PO₄, pH 6.0, 137 mM NaCl, 2.7 mM KCI, 2 mM MgCl₂, 1 mM CaCl₂, 0.01% Tween 20) served as binding and washing buffer during the assay. The target molecule is a chemotherapeutic used for cancer treatment (Imatinib). Stock solutions of 1 mg/mL were prepared in DMSO and stored until usage at -20°C.

### Biolayer Interferometry (BLI) Measurements

BLI is a label-free technology for measuring biomolecular interactions. It is an optical analytical technique that analyses changes in the interference pattern of white light reflected from two surfaces: a layer of immobilised ligand on the biosensor tip, and an internal reference layer. Any change in the number of molecules bound to the biosensor tip causes a shift in the interference pattern that can be measured in real-time. Only molecules binding to or dissociating from the biosensor can shift the interference pattern and generate a response profile on the BLI sensor. Unbound molecules, changes in the refractive index of the surrounding medium, or changes in flow rate do not affect the interference pattern. The Displacement selection principle offers the possibility to develop detection assays based on the duplex formation between the immobilisation sequence of the selected aptamers and the immobilisation oligonucleotide. The target-dependent conformational change leads to the release of the aptamer from the duplex structure. This switch from the hybridised duplex to the displaced stage of the aptamer can be used to generate a recordable signal. The experiments described here were conducted using either the BLItz or Octet QK instruments (ForteBio, Pall Life Sciences, USA).

### Aptamer Displacement Assay by BLI

For aptamer immobilisation onto biosensor probes (Streptavidin-SA Dip&Read Biosensors, ForteBio, Pall Life Sciences, USA), 1.5 µM aptamer and 1 µM immobilisation oligonucleotide were pre-hybridised in buffer BB by heating the mixture to 95°C for 10 minutes and immediately cooling down to 4°C for 5 minutes before mixed with 2x B&W buffer in a 1:1 ratio. Streptavidin coated probes were incubated with this pre-hybridisation mixture for 5 minutes. Three washing steps (30 sec, 120 sec, 30 sec) with buffer PBS6 were performed to remove loosely immobilised DNA material. The probes were then incubated with target solution (20 µM in PBS6) for 5 min. Target binding causes an aptamer displacement which is seen as decrease in signal. The eluted Aptamer-target complexes were recovered for subsequent steps in the 're-capture assay'.

### Re-capture of aptamer-target complexes, assayed by BLI

The biotinylated capture oligonucleotides 'revFOR' and `REV' were immobilised onto separate biosensor probes by incubating a streptavidin-coated probe, for 3 minutes with oligonucleotides revFOR and REV (1 µM in buffer B&W), respectively. Three washing steps (30 sec, 60 sec, 30 sec) with buffer PBS6 were performed to remove loosely immobilised oligonucleotide material. The probes were then incubated for 5 min with the target-eluted material (Aptamer-target complexes) obtained from the Aptamer Displacement Assay (see above). A positive response is recorded as the aptamer-target complexes are re-captured on the immobilised capture oligonucleotides.

### EXAMPLE 2: Microtiter plate-based fluorescence assay using aptamers to detect small molecules in different matrices/substrates (ELISA-like Assay)

### Oligonucleotides and Aptamers:

The assay was performed using nucleic acid molecules (DNA aptamers) specific for the antibiotic Moxifloxacin.

The aptamer comprises the same first and second primer sites as the imatinib aptamer described above and are used for amplification of the aptamer and also used for recapture of the displaced aptamer. The aptamer also includes a nucleic acid sequence capable of binding to at least a portion of immobilisation sequence.

The immobilisation oligonucleotide (complementary to part of the aptamer) comprises a 5' Biotin label and an internal HEGL spacer ISP18. The immobilisation oligonucleotide was HPLC purified prior to use and also manufactured by IDT, Belgium. For the assay, the aptamer is immobilised on to streptavidin-coated microtiter plates (MTP) via the immobilisation oligonucleotide. The immobilisation oligonucleotide contains a defined region of 12 nucleotides (GATCGAGCCTCA, SEQ ID NO: 2) which is complementary to a region of the aptamer which enables hybridisation between both molecules. In addition, the immobilisation oligonucleotide carries a 5' biotin bound via a hexaethylene glycol (HEGL) residue, which is responsible for the coupling of the immobilisation oligonucleotide to the streptavidin-modified MTPs. To re-capture aptamer-target complexes, capture oligonucleotide revFOR was used. The capture oligonucleotide contains defined regions of 19 nucleotides which is complementary to the first region of the aptamer (SEQ ID NO:3) and enables hybridisation between aptamer and capture oligonucleotide (revFOR: was 19 nucleotides in length and comprises a 5' Biotin label. The capture oligonucleotide was HPLC purified prior to use and was manufactured by IDT, Belgium). In alternative embodiments, the capture oligonucleotide comprises a sequence as set forth in SEQ. ID. No. 4.

### Buffers, Target, Matrices/Substrates

Buffer BB (20 mM Tris-HCl pH 7.6, 100 mM NaCl, 2 mM MgCl₂, 1 mM CaCl₂, 0.01% Tween 20) served as binding, washing and hybridisation buffer during the assay. Buffer B&W (5 mM Tris-HCl pH 7.5, 0.5 mM EDTA, 1 M NaCl, 0.01% Tween 20) was used to immobilise biotinylated oligonucleotides onto streptavidin-coated surfaces. The target molecule, the antibiotic was obtained from Sigma-Aldrich, USA. Stock solutions of 5 mM antibiotic were prepared in DMSO and stored until usage at -20°C. Tests were performed in 4 different substrates/matrices: human plasma (HUMANPL32NCU2N,BiolVT, UK); milk (organic whole fat milk, Sainsbury's Supermarket), river water - filter sterilised, filter 0.22 um, ESF-PV-25-022, CM Scientific, UK); mock urine (151.5 mM urea, 64 mM NaCl, 30 mM NaH₂PO₄, 8.85 mM creatinine, 50 mg/L BSA).

### Fluorescence Measurements

Fluorescent markers incorporated into the aptamers allow quantification of the aptamer DNA after different steps of the process, by means of a fluorescence plate reader assay.

Fluorescence measurements of fluorescein (FAM)-labelled DNA were conducted in BMG Fluorescence Plate Reader (FLUOstar OPTIMA, BMG, UK) using the following measuring conditions: excitation 485 nm/emission 520 nm.

### Aptamer Displacement Assay (Microtiter plate-based fluorescence assay)

For aptamer immobilisation onto streptavidin-coated MTPs (Pierce Streptavidin Coated, HBC, Black 96-Well Plates with SuperBlock Blocking Buffer, Thermo Scientific, USA), 0.75 µM aptamer and 0.5 µM immobilisation oligonucleotide were pre-hybridised in buffer BB by heating the mixture to 95°C for 10 minutes and immediately cooling down to 4°C for 5 minutes before being mixed with 2x B&W buffer in an 1:1 ratio. Microtiter plate MTP 1 was incubated with this pre-hybridisation mixture for 1 h at room temperature while shaking at 1000 rpm at MTP shaker (IKA Schüttler MTS 4, IKA Werke GmbH & Co. KG, Germany). Immobilisation efficiency was determined by comparing input and output fluorescence pre and post incubation respectively. This allows calculation of the approximate amount of aptamer loaded by fluorescence measurements.

The aptamer loaded plate (MTP 1) was extensively washed with buffer BB to remove loosely immobilised DNA before incubated for 1 h at room temperature (1000 rpm at MTP shaker) with a gradient of target antibiotic 200 µM, 40 µM, 8 µM, 1.6 µM, 0.32 µM, 0 µM) prepared in 4 different matrices (plasma, milk, river water and mock urine) at 4 concentrations (0%, 10%, 25% & 40% v/v matrix in buffer BB). Target-eluted material (from MTP 1) was recovered and amount of target-binding aptamer was determined by fluorescence measurements.

### Results

The corrected data for both plasma and milk show good antibiotic concentration dependant responses. An element of potentially non-specific binding at the higher matrix concentrations was also observed. This does not affect the validity of the assay or data; but indicates that the correct calibration standard would be required if the assay is to be used to accurately quantify residues in these matrices. The data also shows almost no signal difference for the different concentrations of river water; suggesting that it has no effect on the assay at all. The corrected data for the mock urine do show an antibiotic dependant signal increase but that the signal is reduced with increasing concentrations of sample. This does not affect the validity of the assay, if the correct calibration standard is prepared.

### Recapture of aptamer-target complexes (Microtiter plate-based fluorescence assay)

Biotinylated capture oligonucleotide 'revFOR' was immobilised onto the streptavidin-coated surface of MTP 2 by incubating for 1 h at room temperature while shaking at 1000 rpm at MTP shaker with 0.5 µM capture oligonucleotide revFOR, in immobilisation buffer. The capture oligonucleotide loaded plate (MTP 2) was extensively washed with buffer BB to remove loosely immobilised DNA. The target-eluted material from MTP 1 was transferred after the Aptamer Displacement Assay (see above) to MTP 2 (immobilised with capture oligonucleotide) and incubated for 1 h at room temperature while shaking at 1000 rpm at MTP shaker. Non-captured material was removed from MTP 2 and the amount of recaptured aptamer was determined by fluorescence measurements (after washing to remove the released aptamers and any remaining matrix).

### Results

The data shows that the aptamers were recaptured in each matrix following the same target-concentration dependent trend as the input material. In plasma and milk, a clear matrix-concentration effect can be seen. Aptamers were recaptured at lower amounts in higher matrix concentrations. This does not affect the validity of the assay or data; it simply shows that the correct calibration standard curves would be required if the assay is to be used to accurately quantify residues in these matrices.

### EXAMPLE 3 - concentration-dependent recapture assay

The assay was performed using biotin-labelled Moxifloxacin aptamer (1500pmol) and re-capture oligonucleotide (revFOR or REV, 3400pmol) as described above.

In Step 1, the re-capture oligonucleotide was immobilized on a 96 well plate by first adding 200µl 1x buffer B&W to each well. Serial dilutions of a biotinylated oligo mix (340u! 5x `B&W' buffer, 3400pmol biotin labelled oligo (Forward or reverse), water to 1700ul) were then added across the wells and the plate incubated on an orbital shaker (1000rpm, room temp, 1 hr).

In a second step, an aptamer gradient was prepared across a 96 well plate at pH 6.8 or pH 7.4 in BB buffer, and input fluorescence determined. The plate of step (1) was washed in 3x 200ul of 1x BB at the suitable pH (6.8 or 7.4). A 100ul mix from each well of the plate of step (2) was added to the corresponding wells of the washed plate of step (3) and the plate incubated on an orbital shaker (1000rpm, room temperature, 1 hour).

The plate of step (4) was recovered and 100u! of unbound material transferred to a fresh 96 well plate and fluorescence of the "unbound" material measured using a BMG plate reader. The plate of step (4) was washed with 3 x 200ul of ice cold 1x BB buffer (pH 6.8, 2 minute incubation), and fluorescence of material remaining on the plate surface determined.

### Results

Data was plotted to demonstrate the effect of increasing pre-immobilised biotinylated oligo on the amount of aptamer bound (fluorescence). Figure 8 shows that both immobilised forward and reverse oligo are capable of capturing the FAM labelled aptamer by either the 5' or 3' ends respectively. Figure 8 also shows that pH of the hybridisation buffer has little effect. Each plot also shows that the plates reach a 'saturation point'. For example, when 50pmol of FAM labelled aptamer is added, increased loading is seen up to 50pmol of biotinylated oligo. Above this, no additional aptamer is bound. 2pmol of input aptamer is insufficient to see a trend.

Data was also plotted to demonstrate the effect increasing amount of aptamer input on the amount of aptamer bound (fluorescence). Again, results show that both immobilised forward and reverse oligo are capable of capturing the FAM labelled aptamer (Figure 7). They also show that pH of the hybridisation buffer has little effect. Each plot also shows that increasing aptamer input leads to increased signal (and hence binding). Again, comparison between data sets shows that the plates reach a 'saturation point'. This is most clearly seen at the top point in each set (50pmol input) where clear saturation is seen at ~50pmol immobilised oligo.

### Conclusions

- Recapture of aptamers is possible in selection buffers at both pH 6.8 and pH 7.4 (most common buffers used for small molecule selection).
- Recapture is concentration dependant and linear when 50pmol biotinylated capture oligo is used per well.
- Additional optimisation may be carried out to improve loading if required.

### EXAMPLE 4 - displacement and re-capture assays

Displacement and recapture assays were combined to demonstrate both components in a single assay. In this experiment, aptamers were hybridised to the immobilisation oligo and immobilised onto the microtitre plate surface (plate A). The aptamers were then displaced from the first plate (A) and then recaptured on the second plate (B) pre-immobilised with biotinylated 'capture oligo'. The same aptamer and target were tested in a variety of common matrices (plasma, river water, milk and urine) to demonstrate the versatility of this platform.

### Results

In this assay the amount of fluorescently labelled aptamer retained on the plate (after target-induced displacement), is quantified and plotted against target concentration (Figure 11). The data shows a clear target concentration dependent loss of fluorescent signal from the first plate (A) showing that the aptamer has been displaced by the target (in each of the different matrices).

The displaced aptamers are then recaptured on the second plate, pre-immobilised with the biotinylated 'capture oligo' (B). Again, concentration dependant increase in signals are seen for each of the matrices, suggesting that the matrix does not interfere with recapture.

### Conclusions

- Aptamer displacement shows concentration dependant response in several different matrices.
- Displaced aptamers can be subsequently recaptured on a second ELISA plate, pre-immobilised with the biotinylated 'capture oligo'.
- The presence of matrix (plasma etc) or the target molecule, do not interfere with the recapture.

Throughout the description and claims of this specification, the words "comprise" and "contain" and variations of them mean "including but not limited to" and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

## Claims

1. Apparatus for detecting the presence, absence or level of a target molecule in a sample, the apparatus comprising:
a) a first region comprising:
i) a support; and
ii) an immobilisation oligonucleotide; and
iii) a nucleic acid molecule which has a binding affinity to a target molecule, wherein the nucleic acid molecule is configured to form a complex with the target molecule,
wherein the immobilisation oligonucleotide is attached directly or indirectly to the support, and further wherein the immobilisation oligonucleotide comprises a nucleic acid sequence which is at least partially complementary to a nucleic acid sequence of the nucleic acid molecule and wherein the nucleic acid molecule is capable of hybridizing to the immobilisation oligonucleotide and further wherein the nucleic acid molecule is capable of being displaced from the complex with the immobilisation oligonucleotide if the target molecule is present in the sample; the apparatus further comprising:
b) a further region comprising:
i) a support; and
ii) a capture oligonucleotide attached directly or indirectly to the support, wherein the capture oligonucleotide comprises a nucleic acid sequence which is at least partially complementary to a nucleic acid sequence of the nucleic acid molecule, wherein the capture oligonucleotide is configured to hybridize to the nucleic acid sequence of the nucleic acid molecule when in complex with the target molecule to capture the nucleic acid-molecule-target molecule complex thereby enabling the presence or level of the target molecule to be detected.

2. Apparatus according to claim 1, which further comprises a detection means for detecting the nucleic acid molecule when in a complex with the capture oligonucleotide,
optionally wherein the nucleic acid molecule comprises at least one fixed nucleic acid sequence region, and wherein the capture oligonucleotide comprises a nucleic acid sequence which is complementary to the fixed nucleic acid sequence region of the nucleic acid molecule,
optionally wherein the nucleic acid molecule comprises a first fixed nucleic acid sequence region and a second nucleic acid sequence region, and wherein optionally the first fixed nucleic acid sequence region is located at a 5' end region of the nucleic acid molecule and the second fixed nucleic acid sequence region is located at a 3' end region of the nucleic acid molecule.

3. Apparatus according to claim 1, wherein the immobilisation oligonucleotide is attached indirectly to the support via a linker molecule, and further wherein the nucleic acid molecule is configured to hybridize to the immobilisation oligonucleotide when the immobilisation oligonucleotide is hybridized to the linker molecule, optionally wherein the apparatus further comprises a detection means for detecting formation of a complex comprising the capture oligonucleotide and the nucleic acid molecule.

4. Apparatus according to any preceding claim, which comprises a lateral flow assay device, enzyme-linked oligonucleotide assay (ELONA) or biolayer interferometry (BLI) and/or wherein the recapture of the target molecule - nucleic acid molecule complex is detected and quantified.

5. Apparatus according to claim 4, wherein:
(i) the first region comprises a sample receiving region;
(ii) the apparatus further comprises a flow path between the first region and the further region, wherein optionally the flow path comprises a membrane, e.g. a nitrocellulose, a polyethylene (PE), a polytetrafluoroethylene (PTFE), a polypropylene (PP), a cellulose acetate (CA), a polyacrylonitrile (PAN), a polyimide (PI), a polysulfone (PS), a polyethersulfone (PES) membrane or an inorganic membrane comprising aluminum oxide (Al2O3), silicon oxide (SiO2) and/or zirconium oxide (ZrO2);
(iii) the first region comprises a sample application zone, wherein optionally the sample application zone comprises a sample application pad; or
(iv) the support of the first region comprises an immobilisation zone which comprises the immobilisation oligonucleotide directly or indirectly attached wherein optionally the immobilisation zone comprises woven or non-woven fibers wherein optionally the non-woven fibers are selected from cellulose fibers, glass fibers, silicon carbide fibers, polymer fibers, animal fibers (e.g. wool, silk), carbon fibers, mineral fibers, and/or micro fibers.

6. Apparatus according to any preceding claim, wherein the first region is provided upstream from the further region,
optionally wherein the further region comprises a capture zone in which the capture oligonucleotide is indirectly or directly attached to the support wherein optionally the further region comprises a plurality of capture zones, each capture zone comprising a capture oligonucleotide directly or indirectly attached to the support, wherein each capture oligonucleotide may be the same or may be different.

7. Apparatus according to any preceding claim, wherein:
(i) the apparatus further comprises a control zone located downstream from the first region, wherein the control zone comprises a further capture oligonucleotide,
wherein optionally the apparatus comprises a plurality of control zones, each control zone comprising a capture oligonucleotide, wherein each capture oligonucleotide is the same or different, optionally wherein each capture oligonucleotide in respective control zones is configured to bind to a different molecule; and/or
(ii) the apparatus further comprises a housing enclosing the first region and the further region wherein optionally the housing further comprises a sample introduction port and further optionally wherein the housing further comprises a window adjacent to the detection zone and optionally the control zone.

8. Apparatus according to any of claims 1 to 3, wherein the support of the first region and/ or the further region is independently selected from a bead, a microtiter or other assay plate, a strip, a membrane, a film, a gel, a chip, a microparticle, a nanoparticle, a nanofiber, a nanotube, a micelle, a micropore, a nanopore, and a biosensor surface
wherein optionally the first region is comprised in a first vessel and the further region is comprised in a further vessel.

9. Apparatus according to claim 8, wherein the solid phase of the first region and the further region are comprised on a strip wherein optionally the strip further comprises a flow path between the first region and the further region,
optionally which further comprises one or more of the following:
a) an absorbance pad,
b) a membrane, e.g. a nitrocellulose membrane;
c) one or more competitor molecules; and
d) one or more control molecules.

10. Apparatus according to claim 8 or 9, which further comprises a vessel configured to accommodate at least one end portion of the strip and the sample wherein optionally the vessel comprises a sample introduction region.

11. Apparatus according to any preceding claim, wherein:
(i) the immobilisation oligonucleotide and/or the capture oligonucleotide is selected from a DNA molecule, a RNA molecule, a mixed DNA/RNA molecule, a modified DNA molecule and a modified RNA molecule;
(ii) which comprises a plurality of immobilisation oligonucleotides, a plurality of capture oligonucleotides and a plurality of nucleic acid molecules;
(iii) the nucleic acid molecule is an aptamer and is optionally selected from a double-stranded aptamer, a single-stranded aptamer and an aptamer which is double-stranded over at least a portion of its length; and/or
(iv) the nucleic acid molecule and/or the immobilisation molecule comprises a detectable label,
wherein optionally the detectable label is selected from a fluorophore, a nanoparticle, a quantum dot, an enzyme, a radioactive isotope, a pre-defined sequence portion, a biotin, a desthiobiotin, a thiol group, an amine group, an azide, an aminoallyl group, a digoxigenin, an antibody, a catalyst, a colloidal metallic particle, a colloidal non-metallic particle, an organic polymer, a latex particle, a nanofiber, a nanotube, a dendrimer, a protein, and a liposome and
further optionally wherein the detectable label is an enzyme, and wherein said enzyme is selected from horseradish peroxidase, alkaline phosphatase, urease and β-galactosidase.

12. Apparatus according to any preceding claim, wherein:
(i) the sample is a biological sample selected from whole blood, leukocytes, peripheral blood mononuclear cells, plasma, serum, sputum, breath, urine, semen, saliva, meningial fluid, amniotic fluid, glandular fluid, lymph fluid, nipple aspirate, bronchial aspirate, synovial fluid, joint aspirate, cells, a cellular extract, stool, tissue, a tissue biopsy, and cerebrospinal fluid;
(ii) the sample is derived from an agricultural or industrial product or by-product, an environmental sample, water, a food product, a sample of a production process, an animal product, a plant product and a bacterial product; and/or
(iii) the target molecule is selected from an organic or inorganic small molecule, a cell, a protein, a peptide, an amino acid, a carbohydrate, a lipid, a virus, a microorganism, a tissue section, an ion, a nucleotide, a nucleotide derivative and a nucleic acid.

13. A method of detecting the presence, absence or amount of a target molecule in a sample, the method comprising:
a) interacting a sample with a complex comprising:
i) an immobilisation oligonucleotide; and
ii) a nucleic acid molecule, wherein the immobilisation oligonucleotide is attached directly or indirectly to a support, the immobilisation oligonucleotide comprising a nucleic acid sequence which is at least partially complementary to a nucleic acid sequence of the nucleic acid molecule and wherein the nucleic acid molecule is capable of hybridizing to a portion of the immobilisation oligonucleotide;
wherein the nucleic acid molecule has a binding affinity to a target molecule, and further wherein the nucleic acid molecule is configured to form a complex with the target molecule;
b) if the target molecule is present in the sample, disassociating the nucleic acid molecule from the complex with the immobilisation oligonucleotide to form a target molecule-nucleic acid molecule complex; and
c) providing a capture oligonucleotide which comprises a nucleic acid sequence which is at least partially complementary to a nucleic acid sequence of the nucleic acid molecule, wherein the nucleic acid molecule is capable of hybridizing to a portion of the capture oligonucleotide; and
d) detecting the presence or absence of the target molecule.

14. A method according to claim 13, further comprising, if a target molecule is present in the sample, forming a target molecule-nucleic acid molecule-capture oligonucleotide complex,
optionally wherein the method further comprises quantifying the amount of target molecule in the sample and/or wherein detection of the target molecule comprises photonic detection, electronic detection, acoustic detection, electro-chemical detection, electro-optic detection, enzymatic detection, chemical detection, biochemical detection or physical detection.

15. A method according to claim 13 or 14, wherein step (a) is performed under conditions effective to allow binding between the target molecule and the nucleic acid molecule,
optionally wherein the step of detecting the presence or absence of the target molecule comprises detecting the hybridization of the nucleic acid molecule to the capture oligonucleotide.

## Patentansprüche

1. Einrichtung zum Detektieren des Vorhandenseins, des Nichtvorhandenseins oder des Niveaus eines Zielmoleküls in einer Probe, wobei die Einrichtung Folgendes umfasst:
a) eine erste Region, umfassend:
i) einen Träger; und
ii) ein Immobilisierungsoligonukleotid; und
iii) ein Nukleinsäuremolekül, das eine Bindungsaffinität zu einem Zielmolekül aufweist, wobei das Nukleinsäuremolekül dazu konfiguriert ist, einen Komplex mit dem Zielmolekül zu bilden,
wobei das Immobilisierungsoligonukleotid direkt oder indirekt an den Träger angelagert ist und wobei ferner das Immobilisierungsoligonukleotid eine Nukleinsäuresequenz umfasst, die mindestens teilweise komplementär zu einer Nukleinsäuresequenz des Nukleinsäuremoleküls ist, und wobei das Nukleinsäuremolekül in der Lage ist, mit dem Immobilisierungsoligonukleotid zu hybridisieren, und wobei ferner das Nukleinsäuremolekül in der Lage ist, aus dem Komplex mit dem Immobilisierungsoligonukleotid verdrängt zu werden, falls das Zielmolekül in der Probe vorhanden ist; wobei die Einrichtung ferner Folgendes umfasst:
b) eine weitere Region, umfassend:
i) einen Träger; und
ii) ein Einfangoligonukleotid, das direkt oder indirekt an den Träger angelagert ist, wobei das Einfangoligonukleotid eine Nukleinsäuresequenz umfasst, die mindestens teilweise komplementär zu einer Nukleinsäuresequenz des Nukleinsäuremoleküls ist, wobei das Einfangoligonukleotid dazu konfiguriert ist, mit der Nukleinsäuresequenz des Nukleinsäuremoleküls zu hybridisieren, wenn es sich im Komplex mit dem Zielmolekül befindet, um den Nukleinsäuremolekül-Zielmolekül-Komplex einzufangen, wodurch ermöglicht wird, dass das Vorhandensein oder das Niveau des Zielmoleküls detektiert wird.

2. Einrichtung nach Anspruch 1, die ferner ein Detektionsmittel zum Detektieren des Nukleinsäuremoleküls umfasst, wenn es sich in einem Komplex mit dem Einfangoligonukleotid befindet,
wobei optional das Nukleinsäuremolekül mindestens eine feste Nukleinsäuresequenzregion umfasst und wobei das Einfangoligonukleotid eine Nukleinsäuresequenz umfasst, die komplementär zu der festen Nukleinsäuresequenzregion des Nukleinsäuremoleküls ist,
wobei optional das Nukleinsäuremolekül mindestens eine erste feste Nukleinsäuresequenzregion und eine zweite Nukleinsäuresequenzregion umfasst und wobei optional die erste feste Nukleinsäuresequenzregion an einer 5'-Endregion des Nukleinsäuremoleküls liegt und die zweite feste Nukleinsäuresequenzregion an einer 3'-Endregion des Nukleinsäuremoleküls liegt.

3. Einrichtung nach Anspruch 1, wobei das Immobilisierungsoligonukleotid indirekt über ein Linkermolekül an den Träger angelagert ist und wobei ferner das Nukleinsäuremolekül dazu konfiguriert ist, mit dem Immobilisierungsoligonukleotid zu hybridisieren, wenn das Immobilisierungsoligonukleotid mit dem Linkermolekül hybridisiert ist,
wobei optional die Einrichtung ferner ein Detektionsmittel zum Detektieren einer Bildung eines Komplexes umfasst, der das Einfangoligonukleotid und das Nukleinsäuremolekül umfasst.

4. Einrichtung nach einem vorhergehenden Anspruch, die eine Lateral-Flow-Assay-Vorrichtung, einen enzymgekoppelten Oligonukleotid-Assay (ELONA) oder eine Bio-Layer-Interferometrie (BLI) umfasst und/oder wobei das Wiedereinfangen des Zielmolekül-Nukleinsäuremolekül-Komplexes detektiert und quantifiziert wird.

5. Einrichtung nach Anspruch 4, wobei:
(i) die erste Region eine Probenaufnahmeregion umfasst,
(ii) die Einrichtung ferner einen Strömungspfad zwischen der ersten Region und der weiteren Region umfasst, wobei optional der Strömungspfad eine Membran umfasst, z. B. eine Membran aus Nitrocellulose, Polyethylen (PE), Polytetrafluorethylen (PTFE), Polypropylen (PP), Celluloseacetat (CA), Polyacrylnitril (PAN), Polyimid (PI), Polysulfon (PS), Polyethersulfon (PES) oder eine anorganische Membran, die Aluminiumoxid (Al2O3), Siliciumoxid (SiO2) und/oder Zirconiumoxid (ZrO2) umfasst,
(iii) die erste Region eine Probenauftragszone umfasst, wobei optional die Probenauftragszone ein Probenauftragspad umfasst, oder
(iv) der Träger der ersten Region eine Immobilisierungszone umfasst, die das direkt oder indirekt angelagerte Immobilisierungsoligonukleotid umfasst, wobei optional die Immobilisierungszone gewebte oder nicht gewebte Fasern umfasst, wobei optional die nicht gewebten Fasern ausgewählt sind aus Cellulosefasern, Glasfasern, Siliciumcarbidfasern, Polymerfasern, tierischen Fasern (z. B. Wolle, Seide), Kohlenstofffasern, Mineralfasern und/oder Mikrofasern.

6. Einrichtung nach einem vorhergehenden Anspruch, wobei die erste Region stromaufwärts der weiteren Region bereitgestellt ist,
wobei optional die weitere Region eine Einfangzone umfasst, in der das Einfangoligonukleotid indirekt oder direkt an den Träger angelagert ist, wobei optional die weitere Region eine Vielzahl von Einfangzonen umfasst, wobei jede Einfangzone ein Einfangoligonukleotid umfasst, das direkt oder indirekt an den Träger angelagert ist, wobei jedes Einfangoligonukleotid gleich sein kann oder unterschiedlich sein kann.

7. Einrichtung nach einem vorhergehenden Anspruch, wobei:
(i) die Einrichtung ferner eine Steuerungszone umfasst, die stromabwärts der ersten Region liegt, wobei die Steuerungszone ein weiteres Einfangoligonukleotid umfasst,
wobei optional die Einrichtung eine Vielzahl von Steuerungszonen umfasst, wobei jede Steuerungszone ein Einfangoligonukleotid umfasst, wobei jedes Einfangoligonukleotid gleich oder unterschiedlich ist, wobei optional jedes Einfangoligonukleotid in jeweiligen Steuerungszonen dazu konfiguriert ist, an ein anderes Molekül zu binden; und/oder
(ii) die Einrichtung ferner ein Gehäuse umfasst, das die erste Region und die weitere Region einschließt, wobei optional das Gehäuse ferner eine Probeneinführungsöffnung umfasst und wobei ferner optional das Gehäuse ferner ein Fenster benachbart zu der Detektionszone und optional der Steuerungszone umfasst.

8. Einrichtung nach einem der Ansprüche 1 bis 3, wobei der Träger der ersten Region und/oder der weiteren Region unabhängig ausgewählt ist aus einer Perle, einer Mikrotiter- oder anderen Assayplatte, einem Streifen, einer Membran, einem Film, einem Gel, einem Chip, einem Mikropartikel, einem Nanopartikel, einer Nanofaser, einem Nanoröhrchen, einer Mizelle, einer Mikropore, einer Nanopore und einer Biosensoroberfläche,
wobei optional die erste Region in einem ersten Gefäß umfasst ist und die weitere Region in einem weiteren Gefäß umfasst ist.

9. Einrichtung nach Anspruch 8, wobei die Festphase der ersten Region und der weiteren Region auf einem Streifen umfasst sind, wobei optional der Streifen ferner einen Strömungspfad zwischen der ersten Region und der weiteren Region umfasst,
die optional ferner eines oder mehrere der Folgenden umfasst:
a) ein Absorptionspad,
b) eine Membran, z. B. eine Nitrocellulosemembran;
c) ein oder mehrere Konkurrenzmoleküle; und
d) ein oder mehrere Steuerungsmoleküle.

10. Einrichtung nach Anspruch 8 oder 9, die ferner ein Gefäß umfasst, das dazu konfiguriert ist, mindestens einen Endabschnitt des Streifens und der Probe unterzubringen, wobei optional das Gefäß eine Probeneinführungsregion umfasst.

11. Einrichtung nach einem vorhergehenden Anspruch, wobei:
(i) das Immobilisierungsoligonukleotid und/oder das Einfangoligonukleotid ausgewählt sind aus einem DNA-Molekül, einem RNA-Molekül, einem gemischten DNA/RNA-Molekül, einem modifizierten DNA-Molekül und einem modifizierten RNA-Molekül,
(ii) die eine Vielzahl von Immobilisierungsoligonukleotiden, eine Vielzahl von Einfangoligonukleotiden und eine Vielzahl von Nukleinsäuremolekülen umfasst,
(iii) das Nukleinsäuremolekül ein Aptamer ist und optional ausgewählt ist aus einem doppelsträngigen Aptamer, einem einzelsträngigen Aptamer und einem Aptamer, das über mindestens einen Abschnitt seiner Länge doppelsträngig ist; und/oder
(iv) das Nukleinsäuremolekül und/oder das Immobilisierungsmolekül eine detektierbare Markierung umfasst,
wobei optional die detektierbare Markierung ausgewählt ist aus einem Fluorophor, einem Nanopartikel, einem Quantenpunkt, einem Enzym, einem radioaktiven Isotop, einem vordefinierten Sequenzabschnitt, einem Biotin, einem Desthiobiotin, einer Thiolgruppe, einer Amingruppe, einem Azid, einer Aminoallylgruppe, einem Digoxigenin, einem Antikörper, einem Katalysator, einem kolloidalen metallischen Partikel, einem kolloidalen nichtmetallischen Partikel, einem organischen Polymer, einem Latexpartikel, einer Nanofaser, einem Nanoröhrchen, einem Dendrimer, einem Protein und einem Liposom, und
wobei ferner optional die detektierbare Markierung ein Enzym ist und wobei das Enzym ausgewählt ist aus Meerrettichperoxidase, alkalischer Phosphatase, Urease und β-Galactosidase.

12. Einrichtung nach einem vorhergehenden Anspruch, wobei:
(i) die Probe eine biologische Probe ist, die ausgewählt ist aus Vollblut, Leukozyten, mononukleären Zellen des peripheren Blutes, Plasma, Serum, Sputum, Atem, Urin, Samenflüssigkeit, Speichel, Flüssigkeit aus den Meningen, Fruchtwasser, Drüsenflüssigkeit, Lymphflüssigkeit, Brustwarzenaspirat, Bronchialaspirat, Synovialflüssigkeit, Gelenkaspirat, Zellen, einem Zellextrakt, Stuhl, Gewebe, einer Gewebebiopsie und Zerebrospinalflüssigkeit;
(ii) die Probe abgeleitet ist aus einem landwirtschaftlichen oder industriellen Produkt oder Nebenprodukt, einer Umweltprobe, Wasser, einem Lebensmittelprodukt, einer Probe eines Produktionsvorgangs, einem tierischen Produkt, einem pflanzlichen Produkt und einem bakteriellen Produkt, und/oder
(iii) das Zielmolekül ausgewählt ist aus einem organischen oder anorganischen kleinen Molekül, einer Zelle, einem Protein, einem Peptid, einer Aminosäure, einem Kohlenhydrat, einem Lipid, einem Virus, einem Mikroorganismus, einem Gewebeschnitt, einem Ion, einem Nukleotid, einem Nukleotidderivat und einer Nukleinsäure.

13. Verfahren zum Detektieren des Vorhandenseins, des Nichtvorhandenseins oder der Menge eines Zielmoleküls in einer Probe, wobei das Verfahren Folgendes umfasst:
a) Wechselwirken einer Probe mit einem Komplex, umfassend:
i) ein Immobilisierungsoligonukleotid, und
ii) ein Nukleinsäuremolekül, wobei das Immobilisierungsoligonukleotid direkt oder indirekt an einen Träger angelagert ist, wobei das Immobilisierungsoligonukleotid eine Nukleinsäuresequenz umfasst, die mindestens teilweise komplementär zu einer Nukleinsäuresequenz des Nukleinsäuremoleküls ist, und wobei das Nukleinsäuremolekül in der Lage ist, mit einem Abschnitt des Immobilisierungsoligonukleotids zu hybridisieren;
wobei das Nukleinsäuremolekül eine Bindungsaffinität zu einem Zielmolekül aufweist und wobei ferner das Nukleinsäuremolekül dazu konfiguriert ist, einen Komplex mit dem Zielmolekül zu bilden;
b) falls das Zielmolekül in der Probe vorhanden ist, Dissoziieren des Nukleinsäuremoleküls aus dem Komplex mit dem Immobilisierungsoligonukleotid, um einen Zielmolekül-Nukleinsäuremolekül-Komplex zu bilden; und
c) Bereitstellen eines Einfangoligonukleotids, das eine Nukleinsäuresequenz umfasst, die mindestens teilweise komplementär zu einer Nukleinsäuresequenz des Nukleinsäuremoleküls ist, wobei das Nukleinsäuremolekül in der Lage ist, mit einem Abschnitt des Einfangoligonukleotids zu hybridisieren; und
d) Detektieren des Vorhandenseins oder des Nichtvorhandenseins des Zielmoleküls.

14. Verfahren nach Anspruch 13, das ferner, falls ein Zielmolekül in der Probe vorhanden ist, Bilden eines Zielmolekül-Nukleinsäuremolekül-Einfangoligonukleotid-Komplexes umfasst,
wobei optional das Verfahren ferner Quantifizieren der Menge des Zielmoleküls in der Probe umfasst und/oder wobei die Detektion des Zielmoleküls photonische Detektion, elektronische Detektion, akustische Detektion, elektrochemische Detektion, elektrooptische Detektion, enzymatische Detektion, chemische Detektion, biochemische Detektion oder physikalische Detektion umfasst.

15. Verfahren nach Anspruch 13 oder 14, wobei Schritt (a) unter Bedingungen durchgeführt wird, die wirksam sind, um eine Bindung zwischen dem Zielmolekül und dem Nukleinsäuremolekül zuzulassen,
wobei optional der Schritt des Detektierens des Vorhandenseins oder des Nichtvorhandenseins des Zielmoleküls Detektieren der Hybridisierung des Nukleinsäuremoleküls mit dem Einfangoligonukleotid umfasst.

## Revendications

1. Appareil de détection de la présence, de l'absence ou du niveau d'une molécule cible dans un échantillon, l'appareil comprenant :
a) une première région comprenant :
i) un support ; et
ii) un oligonucléotide d'immobilisation ; et
iii) une molécule d'acide nucléique qui comporte une affinité de liaison avec une molécule cible, dans lequel la molécule d'acide nucléique est configurée pour former un complexe avec la molécule cible,
dans lequel l'oligonucléotide d'immobilisation est attaché directement ou indirectement au support, et en outre dans lequel l'oligonucléotide d'immobilisation comprend une séquence d'acide nucléique qui est au moins partiellement complémentaire d'une séquence d'acide nucléique de la molécule d'acide nucléique et dans lequel la molécule d'acide nucléique est capable de s'hybrider à l'oligonucléotide d'immobilisation et, en outre, dans lequel la molécule d'acide nucléique est capable d'être déplacée du complexe avec l'oligonucléotide d'immobilisation si la molécule cible est présente dans l'échantillon ; l'appareil comprenant en outre :
b) une autre région comprenant :
i) un support ; et
ii) un oligonucléotide de capture attaché directement ou indirectement au support, dans lequel l'oligonucléotide de capture comprend une séquence d'acide nucléique qui est au moins partiellement complémentaire d'une séquence d'acide nucléique de la molécule d'acide nucléique, dans lequel l'oligonucléotide de capture est configuré pour s'hybrider à la séquence d'acide nucléique de la molécule d'acide nucléique lorsqu'elle est en complexe avec la molécule cible pour capturer le complexe acide nucléique-molécule-molécule cible, permettant ainsi de détecter la présence ou le niveau de la molécule cible.

2. Appareil selon la revendication 1, qui comprend en outre un moyen de détection pour détecter la molécule d'acide nucléique lorsqu'elle est dans un complexe avec l'oligonucléotide de capture,
éventuellement dans lequel la molécule d'acide nucléique comprend au moins une région de séquence d'acide nucléique fixée, et dans lequel l'oligonucléotide de capture comprend une séquence d'acide nucléique qui est complémentaire de la région de séquence d'acide nucléique fixée de la molécule d'acide nucléique,
éventuellement, dans lequel la molécule d'acide nucléique comprend une première région de séquence d'acide nucléique fixée et une seconde région de séquence d'acide nucléique, et dans lequel éventuellement la première région de séquence d'acide nucléique fixée est située au niveau d'une région d'extrémité 5' de la molécule d'acide nucléique et la seconde région de séquence d'acide nucléique fixée est située au niveau d'une région d'extrémité 3' de la molécule d'acide nucléique.

3. Appareil selon la revendication 1, dans lequel l'oligonucléotide d'immobilisation est attaché indirectement au support via une molécule de liaison, et en outre dans lequel la molécule d'acide nucléique est configurée pour s'hybrider à l'oligonucléotide d'immobilisation lorsque l'oligonucléotide d'immobilisation est hybridé à la molécule de liaison,
éventuellement, dans lequel l'appareil comprend en outre un moyen de détection pour détecter la formation d'un complexe comprenant l'oligonucléotide de capture et la molécule d'acide nucléique.

4. Appareil selon l'une quelconque des revendications précédentes, qui comprend un dispositif de dosage à flux latéral, un dosage oligoenzymatique sur support solide (ELONA) ou une interférométrie en biocouche (BLI) et/ou dans lequel la recapture du complexe molécule cible - molécule d'acide nucléique est détectée et quantifiée.

5. Appareil selon la revendication 4, dans lequel :
(i) la première région comprend une région de réception d'échantillon ;
(ii) l'appareil comprend en outre un chemin d'écoulement entre la première région et l'autre région, dans lequel éventuellement le chemin d'écoulement comprend une membrane, par ex. une nitrocellulose, un polyéthylène (PE), un polytétrafluoroéthylène (PTFE), un polypropylène (PP), un acétate de cellulose (CA), un polyacrylonitrile (PAN), un polyimide (PI), un polysulfone (PS), une membrane polyéthersulfone (PES) ou une membrane inorganique comprenant de l'oxyde d'aluminium (Al2O3), de l'oxyde de silicium (SiO2) et/ou de l'oxyde de zirconium (ZrO2) ;
(iii) la première région comprend une zone d'application d'échantillon, dans lequel éventuellement la zone d'application d'échantillon comprend un tampon d'application d'échantillon ; ou
(iv) le support de la première région comprend une zone d'immobilisation qui comprend l'oligonucléotide d'immobilisation directement ou indirectement attaché dans lequel éventuellement la zone d'immobilisation comprend des fibres tissées ou non tissées dans lequel éventuellement les fibres non tissées sont choisies parmi des fibres de cellulose, fibres de verre, fibres de carbure de silicium, fibres polymères, fibres animales (par exemple laine, soie), fibres de carbone, fibres minérales et/ou microfibres.

6. Appareil selon l'une quelconque des revendications précédentes, dans lequel la première région est prévue en amont de l'autre région,
éventuellement, dans lequel l'autre région comprend une zone de capture dans laquelle l'oligonucléotide de capture est indirectement ou directement attaché au support, dans lequel éventuellement l'autre région comprend une pluralité de zones de capture, chaque zone de capture comprenant un oligonucléotide de capture directement ou indirectement attaché au support, dans lequel chaque oligonucléotide de capture peut être identique ou différent.

7. Appareil selon l'une quelconque des revendications précédentes, dans lequel :
(i) l'appareil comprend en outre une zone de contrôle située en aval de la première région, dans lequel la zone de contrôle comprend un autre oligonucléotide de capture,
dans lequel éventuellement l'appareil comprend une pluralité de zones de contrôle, chaque zone de contrôle comprenant un oligonucléotide de capture, dans lequel chaque oligonucléotide de capture est identique ou différent, éventuellement dans lequel chaque oligonucléotide de capture dans les zones de contrôle respectives est configuré pour se lier à une molécule différente ; et/ou
(ii) l'appareil comprend en outre un boîtier renfermant la première région et l'autre région, dans lequel éventuellement le boîtier comprend en outre un port d'introduction d'échantillon et en outre éventuellement dans lequel le boîtier comprend en outre une fenêtre adjacente à la zone de détection et éventuellement à la zone de contrôle.

8. Appareil selon l'une quelconque des revendications 1 à 3, dans lequel le support de la première région et/ou de l'autre région est choisi indépendamment parmi une bille, une microtitrage ou une autre plaque de dosage, une bandelette, une membrane, un film, un gel, une puce, une microparticule, une nanoparticule, une nanofibre, un nanotube, une micelle, un micropore, un nanopore et une surface de biocapteur
dans lequel éventuellement, la première région est comprise dans un premier récipient et l'autre région est comprise dans un autre récipient.

9. Appareil selon la revendication 8, dans lequel la phase solide de la première région et de l'autre région sont comprises sur une bandelette dans lequel éventuellement la bandelette comprend en outre un chemin d'écoulement entre la première région et l'autre région,
éventuellement, qui comprend en outre un ou plusieurs des éléments suivants :
a) un tampon absorbant,
b) une membrane, par ex. une membrane de nitrocellulose ;
c) une ou plusieurs molécules concurrentes ; et
d) une ou plusieurs molécules de contrôle.

10. Appareil selon la revendication 8 ou 9, qui comprend en outre un récipient configuré pour recevoir au moins une partie d'extrémité de la bandelette et de l'échantillon, dans lequel éventuellement le récipient comprend une région d'introduction d'échantillon.

11. Appareil selon l'une quelconque des revendications précédentes, dans lequel :
(i) l'oligonucléotide d'immobilisation et/ou l'oligonucléotide de capture sont choisis parmi une molécule d'ADN, une molécule d'ARN, une molécule mixte d'ADN/ARN, une molécule d'ADN modifiée et une molécule d'ARN modifiée ;
(ii) qui comprend une pluralité d'oligonucléotides d'immobilisation, une pluralité d'oligonucléotides de capture et une pluralité de molécules d'acide nucléique ;
(iii) la molécule d'acide nucléique est un aptamère et est éventuellement choisie parmi un aptamère double brin, un aptamère simple brin et un aptamère double brin sur au moins une partie de sa longueur ; et/ou
(iv) la molécule d'acide nucléique et/ou la molécule d'immobilisation comprennent un marqueur détectable,
dans lequel éventuellement le marqueur détectable est choisi parmi un fluorophore, une nanoparticule, un point quantique, une enzyme, un isotope radioactif, une partie de séquence prédéfinie, une biotine, une desthiobiotine, un groupe thiol, un groupe amine, un azide, un groupe aminoallyle, une digoxigénine, un anticorps, un catalyseur, une particule métallique colloïdale, une particule colloïdale non métallique, un polymère organique, une particule de latex, une nanofibre, un nanotube, un dendrimère, une protéine et un liposome et
en outre, éventuellement, dans lequel le marqueur détectable est une enzyme, et dans lequel ladite enzyme est choisie parmi la peroxydase de raifort, la phosphatase alcaline, l'uréase et la β-galactosidase.

12. Appareil selon l'une quelconque des revendications précédentes, dans lequel :
(i) l'échantillon est un échantillon biologique choisi parmi le sang total, les leucocytes, les cellules mononucléées du sang périphérique, le plasma, le sérum, le crachat, l'haleine, l'urine, le sperme, la salive, le liquide méningé, le liquide amniotique, le liquide glandulaire, le liquide lymphatique, l'aspiration du mamelon, l'aspiration bronchique, le liquide synovial, l'aspiration articulaire, des cellules, un extrait cellulaire, des selles, des tissus, une biopsie tissulaire et du liquide céphalo-rachidien ;
(ii) l'échantillon provient d'un produit ou sous-produit agricole ou industriel, d'un échantillon environnemental, d'eau, d'un produit alimentaire, d'un échantillon d'un processus de production, d'un produit animal, d'un produit végétal et d'un produit bactérien ; et/ou
(iii) la molécule cible est choisie parmi une petite molécule organique ou inorganique, une cellule, une protéine, un peptide, un acide aminé, un glucide, un lipide, un virus, un micro-organisme, une coupe de tissu, un ion, un nucléotide, un dérivé nucléotidique et un acide nucléique.

13. Procédé de détection de la présence, de l'absence ou de la quantité d'une molécule cible dans un échantillon, le procédé comprenant :
a) l'interaction d'un échantillon avec un complexe comprenant :
i) un oligonucléotide d'immobilisation ; et
ii) une molécule d'acide nucléique, dans lequel l'oligonucléotide d'immobilisation est attaché directement ou indirectement à un support, l'oligonucléotide d'immobilisation comprenant une séquence d'acide nucléique qui est au moins partiellement complémentaire d'une séquence d'acide nucléique de la molécule d'acide nucléique et dans lequel la molécule d'acide nucléique est capable de s'hybrider à une partie de l'oligonucléotide d'immobilisation ;
dans lequel la molécule d'acide nucléique comporte une affinité de liaison pour une molécule cible, et en outre dans lequel la molécule d'acide nucléique est configurée pour former un complexe avec la molécule cible ;
b) si la molécule cible est présente dans l'échantillon, la dissociation de la molécule d'acide nucléique du complexe avec l'oligonucléotide d'immobilisation pour former un complexe molécule cible-molécule d'acide nucléique ; et
c) la fourniture d'un oligonucléotide de capture qui comprend une séquence d'acide nucléique qui est au moins partiellement complémentaire d'une séquence d'acide nucléique de la molécule d'acide nucléique, dans lequel la molécule d'acide nucléique est capable de s'hybrider à une partie de l'oligonucléotide de capture ; et
d) la détection de la présence ou l'absence de la molécule cible.

14. Procédé selon la revendication 13, comprenant en outre, si une molécule cible est présente dans l'échantillon, la formation d'un complexe molécule cible-molécule d'acide nucléique-oligonucléotide de capture,
éventuellement, dans lequel le procédé comprend en outre la quantification de la quantité de molécule cible dans l'échantillon et/ou dans lequel la détection de la molécule cible comprend une détection photonique, une détection électronique, une détection acoustique, une détection électrochimique, une détection électro-optique, une détection enzymatique, une détection chimique, une détection biochimique ou une détection physique.

15. Procédé selon la revendication 13 ou 14, dans lequel l'étape (a) est réalisée dans des conditions efficaces pour permettre la liaison entre la molécule cible et la molécule d'acide nucléique,
éventuellement dans lequel l'étape de détection de la présence ou de l'absence de la molécule cible comprend la détection de l'hybridation de la molécule d'acide nucléique à l'oligonucléotide de capture.
